(19) Europäisches Patentamt
European Patent Office
Office européen des brevets

(11) EP 1 203 047 B1

(12) **FASCICULE DE BREVET EUROPEEN**

(45) Date de publication et mention de la délivrance du brevet:
**09.01.2008 Bulletin 2008/02**

(21) Numéro de dépôt: **00956636.5**

(22) Date de dépôt: **07.08.2000**

(51) Int Cl.:
***C08G 83/00*** *(2006.01)* ***C12Q 1/68*** *(2006.01)*
***C07D 207/32*** *(2006.01)* ***C07D 209/16*** *(2006.01)*
***C07C 235/84*** *(2006.01)*

(86) Numéro de dépôt international:
**PCT/FR2000/002267**

(87) Numéro de publication internationale:
**WO 2001/012699 (22.02.2001 Gazette 2001/08)**

(54) **ELECTROPOLYMERES PHOTOGREFFABLES, LEUR PROCEDE D'OBTENTION ET LEURS APPLICATIONS COMME SUPPORTS DE SONDES DE RECONNAISSANCE SPECIFIQUE DANS DES BIOCAPTEURS ELECTRONIQUES**

PHOTO-PFROPFUNGS-ELEKTROPOLYMERE, IHRE HERSTELLUNG UND DEREN VERWENDUNG ALS TRÄGER FÜR ERKENNUNGSSONDEN SPEZIFISCH IN BIOSENSORSYSTEMEN

ELECTRICALLY CONDUCTIVE POLYMERS CAPABLE OF BEING COVALENTLY GRAFTED ON BY LIGHT, METHOD FOR OBTAINING SAME AND USES AS SUPPORTS IN PROBES FOR SPECIFIC IDENTIFICATION IN ELECTRONIC BIOSENSORS

(84) Etats contractants désignés:
**AT BE CH CY DE DK ES FI FR GB GR IE IT LI LU MC NL PT SE**

(30) Priorité: **12.08.1999 FR 9910535**
**29.11.1999 FR 9915022**

(43) Date de publication de la demande:
**08.05.2002 Bulletin 2002/19**

(73) Titulaire: **UNIVERSITE JOSEPH FOURIER, UJF-INDUSTRIE**
**F-38041 Grenoble Cédex 9 (FR)**

(72) Inventeur: **COSNIER, Serge**
**F-38920 Crolles (FR)**

(74) Mandataire: **Sarlin, Laure V. et al**
**Cabinet Beau de Loménie,**
**51, avenue Jean-Jaurès,**
**BP 7073**
**69301 Lyon Cedex 07 (FR)**

(56) Documents cités:
**WO-A-90/10655** **WO-A-95/29199**

- **DATABASE WPI Section Ch, Week 199047 Derwent Publications Ltd., London, GB; Class A26, AN 1990-352848 XP002139597 & JP 02 255716 A (RICOH KK), 16 octobre 1990 (1990-10-16)**
- **DATABASE WPI Section Ch, Week 199520 Derwent Publications Ltd., London, GB; Class A26, AN 1995-151827 XP002139598 & JP 07 076572 A (OSAKA GAS CO LTD), 20 mars 1995 (1995-03-20)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

Le domaine de l'invention est celui des électropolymères et en particulier de leur application comme matériaux constitutifs de biocapteurs et de capteurs électroanalytiques.

La présente invention concerne des polymères obtenus par électropolymérisation également dénommés électropolymères, ainsi que leur procédé de préparation. Les monomères et/ou oligomères impliqués dans ce procédé sont également compris dans la présente invention. Cette dernière vise également l'utilisation de ces électropolymères comme supports d'ancrage de sondes de reconnaissance spécifique faisant partie de biocapteurs ou de capteurs électroanalytiques e.g. : (micro)électrodes, capteurs à transistor à effet de champ, biopuces, semi-conducteurs, fibres optiques conductrices...

La combinaison des propriétés de reconnaissance moléculaire spécifique voire stéréospécifique des macromolécules biologiques avec l'extrême sensibilité de capteurs optiques, électrochimiques ou gravimétriques a conduit à l'émergence d'une nouvelle génération d'outils analytiques : les biocapteurs. Depuis trois décennies, le développement des biocapteurs connaît une expansion majeure en raison de leurs potentielles applications industrielles dans le domaine de l'environnement (dosage in situ de polluants) et de l'analyse biomédicale (biocapteur portable ou à usage unique, remplacement des tests ELISA). Récemment, l'essor des technologies en microélectronique a offert de nouvelles opportunités pour la miniaturisation des biocapteurs. Les objectifs principaux sont les biocapteurs implantables ou l'association de plusieurs biocapteurs afin d'obtenir simultanément des informations sur plusieurs analytes. Ainsi, des microcapteurs peuvent être fabriqués par immobilisation de biomolécules à la surface d'une large variété de microtransducteurs tels que les multiplots de microélectrode, les microélectrodes interdigités ou les transistors à effet de champ.

Ces biomolécules (oligo- ou polynucléotides-ADN-ARN, enzymes, anticorps, antigènes, Acides Nucléiques Peptidiques, oligo ou polypeptides) se comportent comme des sondes de reconnaissance spécifique, par exemple par interaction affine de substances biologiques cibles à analyser. La référence à la biologie pour qualifier les molécules concernées n'est pas exclusive d'autres molécules analogues aux molécules d'origine biologique stricto sensu, mais fabriquées par voie chimique synthétique (e.g. chimères).

La fabrication de microbiocapteurs est actuellement l'enjeu d'une âpre compétition internationale, la réalisation de biopuces pour le séquençage de l'ADN en étant la parfaite illustration. L'élaboration de plusieurs milliers de "spots" d'oligonucléotides différents à des positions parfaitement localisées sur un support plan peut être utilisée pour identifier et quantifier des ADN ou ARN fonctionnalisés par des marqueurs fluorescents.

Toutefois, un verrou technologique demeure dans l'élaboration des microbiocapteurs. En effet, l'immobilisation stable, reproductible et spatialement contrôlée de sondes de reconnaissance spécifique (par exemple d'oligonucléotides , de polyacide nucléiques peptidiques ou de protéines) avec rétention totale de leurs propriétés biologiques reste un problème crucial. Effectivement, la plupart des procédures conventionnelles d'immobilisation de sondes e.g. protéiques, comme la réticulation, le greffage covalent ou le piégeage dans des gels ou des membranes souffre d'une faible reproductibilité et d'une mauvaise résolution spatiale.

D'autres approches d'immobilisation de sondes par exemple protéiques, ont été développées à partir de technologies conventionnelles d'impression comme le "screen printing" et le "ink-jet deposition" permettant une localisation précise de la protéine. Toutefois, l'immobilisation par piégeage de la protéine peut réduire l'accessibilité de cette dernière et donc diminuer l'efficacité des interactions spécifiques du type oligonucléotides complémentaires, anticorps-antigène ou enzyme-substrat.

Plus récemment, une autre méthode a mis en jeu le dépôt contrôlé de microvolumes (5nl) contenant la protéine à dés emplacements précis. Dans cette approche, les problèmes rencontrés sont des problèmes d'évaporation nécessitant de travailler dans des pièces à humidité contrôlée et des problèmes de contamination d'un dépôt à un autre.

Il est également connu de recourir à la photolithographie de groupes photoactivables pour immobiliser des biomolécules sur des supports compris dans des microtransducteurs de biocapteurs.

C'est ainsi que l'article de L.F. Rozsnyai, D.R. Benson, S.P.A. Fodor et P.G. Schultz, Angew, Chem. Int. Ed. Engl. 31 (1992) 759, décrit l'ancrage covalent d'anticorps sur un support en silice fonctionnalisé par des motifs photoactivables du type benzophénone. La fonctionnalisation (ou dérivatisation) des substrats en silice est réalisée à l'aide de 3-aminopropyltriéthoxysilane protégé par des groupements t-butyloxycarbonyl(Boc). Après déprotection du silane de surface, un ester N-hydroxysuccinimide de l'acide 3-benzoylbenzoïque est couplé au silane de surface. On dépose ensuite les anticorps à greffer sur le support et on expose ces derniers, au travers d'un masque, à un rayonnement lumineux qui activera dans les zones découvertes la liaison de l'immunoglobuline aux carbonyles des motifs benzophénones fixés sur le support.

La demande de brevet internationale PCT WO.92/10092 décrit une technique de synthèse in situ d'un réseau d'oligonucléotides sur un substrat silicium silanisé à l'aide de 3-aminopropyltriéthoxysilane et protégé par un groupement photosensible du type 6-nitrovératryoxycarbonyl (NVOC). Selon cette technique on réalise une déprotection localisée du substrat par exposition à la lumière au travers d'un masque. La zone ou les zones déprotégées sont ensuite le siège de réactions de couplage chimique soit des nucléotides entre eux pour former des oligonucléotides soit des nucléotides

avec le support pour fixer sur celui-ci des oligonucléotides synthétisés ex-situ à des nucléotides de tête dans le cadre de la synthèse in situ d'oligonucléotides:

Dans ces techniques photolithographiques de greffage de sondes sur support, on contrôle la répartition géométrique desdites sondes grâce à des photomasques mais les inconvénients majeurs de ces techniques résident dans l'absence de contrôle du dépôt des motifs photoactivables sur le support.

Une alternative à ces méthodes consiste à immobiliser des sondes de reconnaissance spécifique, par exemple de nature protéique, par électropolymérisation d'un monomère, en présence des sondes protéiques à immobiliser. Cela conduit à l'encagement des sondes protéiques dans des films polymères électrogénérés. La localisation des sondes peut être spatialement contrôlée grâce aux possibilités d'adressage électrochimique du film polymère qu'offre la méthode. A titre d'illustration, on peut citer l'article de Serge Cosnier (Can J. Chem. Eng., 76 (1998) 1000) qui concerne la fabrication de biocapteurs ampérométriques par encagement dans des films de polypyrrole fonctionnalisé. La procédure électrochimique d'immobilisation d'enzymes décrite dans cet article comprend deux étapes. La première consiste en l'adsorption d'un mélange aqueux d'enzyme et de pyrrole amphiphile sur une surface d'électrode et la seconde étape est l'électropolymérisation des monomères adsorbés.

L'encagement d'enzymes dans des électropolymères est également illustré par le brevet américain N° 5 286 364 qui divulgue l'électropolymérisation de 1,3-diaminobenzène et de résorcinol. L'enzyme immobilisée dans l'électropolymère généré de manière localisée, est la glucose-oxydase.

Cette technique d'encagement de substances sensibles (sondes) de nature protéique dans des films polymères électrogénérés, présente l'inconvénient de réduire radicalement l'accessibilité à la substance immobilisée. En particulier, les contraintes stériques générées par le polymère entourant les sondes protéiques ou oligonucléotidiques , peuvent entraver la formation des interactions spécifiques de reconnaissance du type antigène-anticorps, enzyme-substrat ou hybridation d'oligonucléotides complémentaires. Par conséquent, l'immobilisation électrochimique de protéines ou d'oligonucléotides ne s'avère pas la méthode la plus appropriée pour la fabrication de biocapteurs.

Une approche légèrement différente consiste à électrogénérer un polymère comportant des groupements susceptibles de générer un couplage covalent avec une protéine. Ainsi des enzymes et des oligonucléotides ont pu être immobilisés sur des polypyrroles par greffage chimique. Toutefois, ces réactions chimiques peuvent dénaturer les substances sondes sensibles (protéines-polynucléotides).

Une stratégie plus prometteuse de fixation de sondes, par exemple protéiques ou oligonucléotidiques, sur des films électrogénérés, est l'immobilisation par l'intermédiaire de systèmes affins, qui préservent totalement l'activité biologique des protéines. Selon cette stratégie, on procède à l'électrogénération d'un film polymère fonctionnalisé par des motifs biotine. En raison des fortes interactions affines entre la biotine (poids moléculaire = 250 D) et l'avidine (poids moléculaire = 66 000 D) une monocouche d'avidine peut se former spontanément à la surface du polymère, par simple immersion du support, en l'occurrence du transducteur, dans une solution aqueuse d'avidine. La fixation ultérieure de sondes biotinylées (enzyme, anticorps, antigène, oligonucléotides, polyacide nucléique peptidique..) intervient également par simple immersion du support, en l'occurrence du transducteur, modifié par l'avidine, dans la solution aqueuse de substance sonde choisie, la constante d'association entre l'avidine et les motifs biotine étant : $K_a = 10^{15} M^{-1}$. L'immobilisation de sondes, par exemples protéiques, est ainsi réalisée par un ancrage non dénaturant conférant une accessibilité maximale à la protéine.

L'inconvénient majeur de cette méthode électroaffine est la nécessité d'utiliser des sondes, en particulier des protéines marquées par des fonctions biotine ou des sondes, en particulier des protéines, conjuguées à des avidines.

A titre d'illustration de ces techniques électroaffines, on renverra aux articles suivants:

- "Electrogeneration of biotinylated functionalized polypyrroles for the simple immobilization of enzymes" S. Cosnier, B. Galland, C. Gondran et A. Le Pellec, Electroanalysis, 10 (1998) 808.
- "Poly(pyrrole-biotine) : a new polymer for biomolecule grafting on electrode surfaces", S. Cosnier et A. Le Pellec, Electrochim. Act, 44 (1999) 1833.

On connaît également des électropolymères sur lesquels sont greffés chimiquement des substances sondes sensibles. Ces électropolymères porteurs de sondes greffées chimiquement ont vocation à être utilisés comme moyens d'immobilisation de sondes ou de marqueurs dans des biocapteurs.

C'est ainsi que la demande de brevet français FR-A-2 703 359 concerne un copolymère électrogéné du type polypyrrole dans lequel certains monomères pyrroles sont substitués par des chaînes latérales pendantes oligonucléotidiques, par l'intermédiaire de rotules d'espacement du type aminoéthyle.

La demande de brevet français FR-A-2 750 136 vise également des polypyrroles électrogénérés greffés, mais cette fois-ci les greffons oligonucléotidiques sont porteurs d'une molécule active (hormone : ACTH ou biotine).

La demande de brevet européen EP-A-314 009 divulgue des polymères électrogénérés du type polydithiénylpyrroles fonctionnalisés par des enzymes telles que la glucose-oxydase. Le greffage du motif fonctionnalisé peut être réalisé par l'intermédiaire d'une amine (protégée ou non) portée par le noyau pyrrole.

La demande PCT WO 90/10 655 décrit des électropolymères du type polyacétylène-cis greffés par des enzymes du type glucose-oxydase, via des rotules d'espacement du genre benzoquinone hydroxylée. Cette demande PCT divulgue également des polyanilines électrogénérées et fonctionnalisées par des indicateurs réactifs du type glucose-oxydase ou cofacteur correcteur enzymatique (FAD).

La demande de brevet français FR-A-2 720 832 est relative à des électropolymères du type polythiophène, polypyrrole, polyphénylène, polythiophène-vinylène, polyaniline, polyacétylène sur lesquels ont été greffés des peptides biologiquement actifs, en vue d'une interaction spécifique avec des espèces chimiques ou biologiques d'intérêt biologique ou médical (hormones par exemple). Ce greffage est réalisé chimiquement par l'intermédiaire d'un bras espaceur acétyle relié à l'atome de carbone n° 3 du noyau pyrrole. Ces électropolymères greffés peuvent être utilisés comme membranes électroactives dans des biocapteurs.

Tous ces électropolymères connus sur lesquels sont greffées chimiquement des molécules sondes sensibles, ont précisément l'inconvénient d'être obtenus par des procédés chimiques susceptibles d'altérer les molécules sondes à greffer. Par ailleurs, il est difficile dans ces procédés de contrôler la localisation des greffons. En conséquence, il n'est pas possible de réaliser des réseaux organisés de sondes différentes de reconnaissance spécifique, intégrables dans des biocapteurs.

Le bilan qui s'impose à l'issue de cette revue de l'état de la technique est donc qu'il existe toujours un besoin aigu en un support polymère susceptible de constituer la membrane sensible de biocapteurs, sachant que les spécifications recherchées pour cette membrane (ou ce revêtement sensible) sont :

- de pouvoir être porteuse de sondes sensibles de reconnaissance spécifique immobilisées de manière fiable et durable,
- de faire intervenir un mode d'immobilisation des sondes sensibles qui ne porte pas préjudice à leur capacité d'interagir spécifiquement avec les molécules cibles à analyser,
- d'être porteuse de sondes sensibles (par exemple protéines, oligonucléotides, polyacides nucléique peptidique.....) qui ne soient pas dénaturées lors de l'immobilisation,
- d'offrir la possibilité de contrôler aisément la localisation des sondes de façon à pouvoir réaliser des réseaux de sondes,
- de pouvoir être obtenue de manière simple et économique de façon à pouvoir être industrialisable.

L'un des objectifs essentiels de la présente invention est de pallier ces carences en fournissant de nouveaux polymères sur lesquels peuvent être greffés aisément des sondes de reconnaissance spécifique, non dénaturées, ayant une capacité optimale de réaction avec les analytes cibles et dont la localisation soit contrôlable, ces nouveaux polymères se devant également d'être d'un coût de revient modéré.

Un autre objectif essentiel de l'invention est de fournir de nouveaux moyens d'immobilisation de sondes spécifiques du type peptide polynucléotide, polyacide nucléique peptidique, permettant une immobilisation stable, économique, non dénaturante et spatialement contrôlée des sondes.

Un autre objectif essentiel de l'invention est de fournir des procédés de préparation des électropolymères susvisés qui sont susceptibles de constituer un support d'immobilisation adapté pour sondes de reconnaissance spécifique pour (micro)biocapteurs électroniques.

Un autre objectif essentiel de l'invention est de fournir de nouveaux monomères à partir desquels les polymères susvisés sont susceptibles d'être obtenus.

Un autre objectif essentiel de l'invention est de proposer un procédé de fabrication de biocapteur faisant intervenir la préparation des électropolymères sus-évoqués.

Un autre objectif essentiel de l'invention est de fournir un biocapteur électronique dont la membrane sensible est constituée par les polymères susvisés sur lesquels sont greffés des sondes de reconnaissance spécifique.

Ces objectifs, parmi d'autres, sont atteints par la présente invention qui concerne en premier lieu de nouveaux monomères comprenant au moins une entité électropolymérisable et au moins un motif à la fois photoactivable et photogreffable, choisis parmi :

■ le monomère pyrrolbenzophénone de formule :

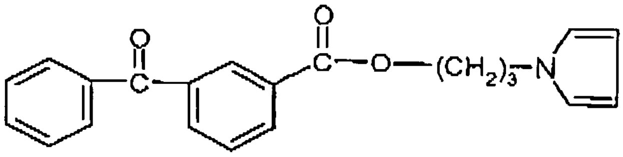

(benzophénone) (pyrrole)

en particulier obtenu par réaction de l'acide 3-benzoylbenzoïque avec le 3-pyrrol-1-propanol,

■ le monomère 4-(2-aminoéthyl)phénol-benzophénone de formule :

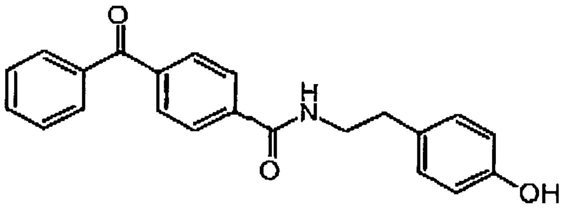

en particulier obtenu par réaction du 4-(2-aminoéthyl)phénol avec l'acide 4-benzoylbenzoïque,

■ le monomère indol-benzophénone de formule :

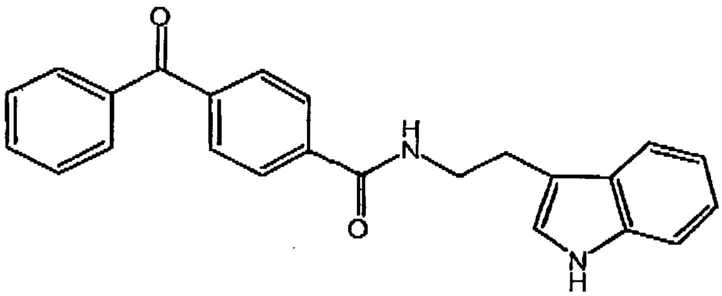

en particulier obtenu par réaction du 3-(2-aminoéthyl)indole avec l'acide 4-benzoylbenzoïque,

■ le monomère vinylaniline-benzophénone de formule :

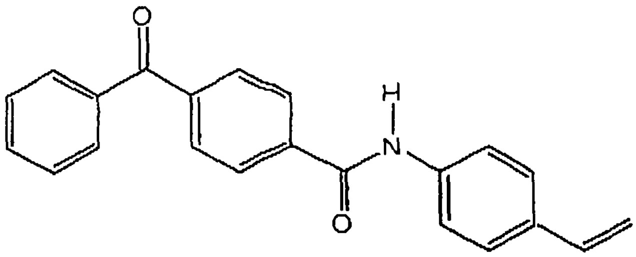

en particulier obtenu par réaction de la vinylaniline avec l'acide 4-beztzoylbenzoïque,

■ le monomère azidophénylpyrrole de formule

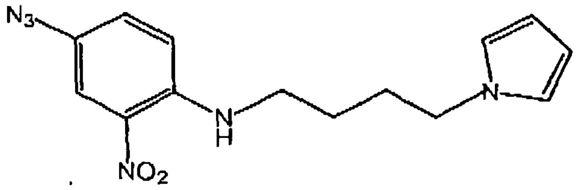

en particulier obtenu par réaction de 4-fluoro-3-nitro-phénylazide avec l'aminobutylpyrrole, ainsi que les électropolymères photogreffables préparés à partir d'un tel monomère.

Les électropolymères selon l'invention peuvent être préparés rapidement et aisément sous forme de films, en contrôlant de manière précise l'épaisseur du film par l'intermédiaire de la charge électrique mise en oeuvre. Ces électropolyméres peuvent être déposés sur toutes sortes de supports et en particulier sur de petites surfaces d'électrodes à géométrie complexe.
Plus précisément au sens de l'invention, les électropolymères sont des films polymères organiques obtenus par oxydation ou réduction électrochimique, à la surface d'une électrode, de monomères soit adsorbés sur cette surface soit solubilisés dans un milieu électrolytique aqueux ou organique.
Les électropolymères ne peuvent se former que sur des surfaces conductrices ou des semi-conducteurs capables de véhiculer la charge électrique nécessaire à la polymérisation. Cette caractéristique permet d'envisager un adressage électrochimique. Cela signifie qu'en alimentant en courant électrique certaines zones du support conducteur, de préférence à d'autres, on peut circonscrire la formation du polymère dans ces zones alimentées et obtenir ainsi des zones différenciées et indépendantes porteuses chacune d'une sonde photogreffée donnée.
Les électropolymères photogreffables selon l'invention sont propres à se lier de manière simple, économique, reproductible et spatialement contrôlée à des sondes de reconnaissance spécifique, qui peuvent être des protéines, des polynucléotides, des polyacides nucléiques peptidiques ou analogues.
Une telle immobilisation par photogreffage permet de s'affranchir du recours à des réactifs chimiques éventuellement dénaturants. De plus, la technique est simple car les sondes n'ont pas besoin d'être prétraitées ou fonctionnalisées pour pouvoir être greffées. Enfin, le greffage peut s'opérer de manière précise et rapide, à l'aide de photomasques.
A propos des motifs à la fois photoactivables et photogreffables des électropolymères selon l'invention, il convient de signaler que lesdits motifs sont, sélectionnés dans le groupe comprenant : les phénylazides, les dérivés de benzophénone.
Conformément à l'invention, la benzophénone est un motif photoactivable particulièrement préféré.
De manière plus générale, on entend au sens de l'invention par motif photoactivable : un groupement chimique qui, sous irradiation, est converti en un composé hautement réactif susceptible de former une liaison covalente avec d'autres composés en solution en milieu soit organique soit aqueux.
Le taux de motifs à la fois photoactivables et photogreffables des électropolymères selon l'invention est un paramètre qui permet de les caractériser.
Ainsi, il est particulièrement avantageux que, selon l'invention, au moins 0,01 % en nombre de ses (co)monomères constitutifs, de préférence au moins 10 % en nombre de ses (co)monomères constitutifs, et plus préférentiellement encore chacun de ses comonomères constitutifs, est (sont) porteur(s) d'au moins un motif à la fois photoactivable et photogreffable.
Naturellement, les électropolymères selon l'invention peuvent être soit des homopolymères soit des copolymères.
Ces copolymères peuvent être de type bloc ou de type statistique
Selon une caractéristique avantageuse de l'invention, les électropolymères photogreffables qu'elle concerne ont un degré de polymérisation $DP_n$ compris entre 200 et $10^7$, de préférence compris entre $10^3$ et $10^7$ et plus préférentiellement encore entre $10^4$ et $10^7$.
Ces électropolymères peuvent également être définis par le nombre de molécules monomériques immobilisées par unité de surface, qui est de préférence compris entre $10^{-11}$ et $10^{-6}$ mole $cm^{-2}$ et, plus préférentiellement encore, entre 5 x $10^{-9}$ et $10^{-8}$ mole.$cm^{-2}$ ; l'épaisseur du film polymère à $10^{-6}$ mole.$cm^{-2}$ est comprise entre 0,2 et 2 $\mu$m.
Les propriétés électrochimiques des polymères selon l'invention sont un autre moyen pour les définir. C'est ainsi que par voltamétrie cyclique, on observe pour les électropolymères selon l'invention, un signal réversible caractéristique d'un système rédox, d'une valeur de la tension avantageusement inférieure ou égale à 0 volt, de préférence inférieure ou égale à -1 volt et plus préférentiellement encore comprise entre -1,5 et -2,5 volts. Ce signal est dû à la présence du motif photoactivable. De plus, ces polymères peuvent être des polymères conducteurs électroniques ; dans

ce cas la quantité de polymère électrogénéré sur un support conducteur pourra être déterminée via l'intégration de la charge électrique correspondant à l'électroactivité du polymère dans la zone de potentiel comprise entre -1V et + 1V.

La mesure de voltamétrie est réalisée par balayage de potentiel entre les valeurs limites de e.g. : 0 et - 2,5 volts. Le milieu liquide conducteur de mesure est, par exemple, constitué par $CH_3CN$ + TBAP 0,1M. La mesure de tension est réalisée par référence à un système $Ag/Ag^+$ 10mM dans $CH_3CN$.

Selon un mode préféré de réalisation de l'invention les électropolymères qu'elle concerne, sont porteurs sur chacun de leurs monomères constitutifs d'un motif photoactivable.

Les motifs photoactivables peuvent être identiques ou différents entre eux sur une même chaîne polymère et/ou d'une chaîne polymère à l'autre. Conformément au mode préféré de réalisation, les motifs photoactivables sont identiques sur toutes les chaînes polymères.

En dessous d'un $DP_n$ de l'ordre de 50, les électropolymères selon l'invention sont de préférence dénommés "oligomères."

S'agissant de leur préparation, le procédé préféré conformément à l'invention est caractérisé en ce qu'il consiste essentiellement :

- à mettre en oeuvre au moins un type de monomères électropolymérisables ;
- à fixer sur au moins une partie des (co)monomères mis en oeuvre, au moins un motif photoactivable par (co) monomère
- à électropolymériser les (co)monomères, cette électropolymérisation étant de préférence réalisée sur un support conducteur en soumettant les (co)monomères à un balayage potentiel et/ou à une électrolyse à potentiel imposé (chronoampérométrie) ou à courant constant (chronopotentiométrie).

Selon que l'on souhaite obtenir des homo ou des copolymères, on utilisera des monomères identiques entre eux ou des comonomères appartenant au moins à deux espèces différentes.

Dans un mode particulièrement préféré de préparation l'électropolymérisation des monomères ou des comonomères fonctionnalisés ou non par au moins un motif photoactivable, est effectuée sur un support conducteur de manière à former un film d'électropolymère sur toute la zone soumise au champ électrique.

Il est parfaitement envisageable, pour un support d'électropolymérisation donné, de n'alimenter en courant électrique que certaines zones sélectionnées du support conducteur. On réalise ainsi un adressage électrochimique qui permet de localiser l'électropolymère photogreffable à des endroits bien précis.

Avantageusement, les (co)monomères copolymérisables mis en oeuvre avec les monomères selon l'invention sont choisis :

☐ dans le sous-groupe des (co)monomères conducteurs comprenant :

- l'acétylène, les pyrroles, les thiophènes, les indoles, les anilines, les azines, les p-phénylènevinylènes, les p-phénylènes, les pyrènes, les furanes, les sélénophènes, les pyrridazines, les carbazoles et leurs mélanges,

☐ et/ou dans le sous groupe des (co)monomères isolants comprenant les phénols, les ortho-phénylènediamine, les dichlorophénolindophénol et leurs mélanges.

Sur le plan de la méthodologie, plusieurs procédures son envisageables conformément à l'invention. Ainsi, l'étape de greffage intervient avant ou pendant et ou après l'étape d'électropolymérisation.

Dans le mode préféré de mise en oeuvre du procédé, on effectue la substitution des motifs photoactivables et photogreffables sur tous les monomères de départ avant d'engager l'électropolymérisation. Cette dernière implique donc des (co)monomères conjugués chacun à au moins un motif, de préférence un motif photoactivable et photogreffable.

Selon une variante, on pourrait réaliser l'électropolymérisation des monomères non substitués par les motifs photoactivables et ne faire intervenir cette substitution que sur le polymère fini.

Naturellement, les deux variantes sus évoquées sont combinables.

La solution qui consisterait à réaliser simultanément l'électropolymérisation et la substitution des motifs photoactivables n'est certes pas la préférée mais néanmoins ne peut être exclue de l'invention.

L'électropolymérisation est, de préférence, réalisée à partir de monomères, mais il peut être également envisagé d'utiliser des oligomères fonctionnalisés ou non par des motifs photoactivables, en tant que produits de départ de l'électropolymérisation, en combinaison ou non avec des monomères (co) monomères.

L'électropolymérisation est avantageusement réalisée, en pratique, dans des conditions de pression et de température ambiante.

Le support mis en oeuvre peut être par exemple : des électrodes métalliques (or, platine, acier inoxydable), des électrodes de carbone vitreux ou de graphite, des électrodes transparentes (verre ou quartz recouvert d'un film d'or,

de $TiO_2$ ou de $SnO_2$) des électrodes volumiques telles que des feutres de carbone, des poudres de carbone ou de graphite, des toiles tissées de métaux comme par exemple des aciers inoxydables, des gels nanoporeux de $TiO_2$.

Un milieu liquide constitué par une solution, une suspension ou une émulsion de (co)monomères, voire d'oligomères, en phase aqueuse ou organique, est versé sur le support. On soumet ensuite ce dernier à des balayages répétitifs de potentiel (par exemple entre - 0,5 V et 1,2 V). Les zones du support ainsi alimentées sont le siège de la formation en surface d'un film d'électropolymère.

La présence du film d'électropolymère peut être caractérisée par voltamétrie cyclique.

L'électropolymère obtenu est substitué par des motifs photoactivables aptes à permettre le photogreffage de biomolécules sensibles ou sondes de reconnaissance spécifique notamment par interaction affine. Le taux de substitution de l'électropolymère en motifs photoactivables permettant le photoancrage est compris entre 0,01 et 100 %.

Les électropolymères, (co)monomères présentés supra et ayant la propriété d'être photogreffables constituent d'autres objets nouveaux et inventifs lorsqu'ils portent des éléments photogreffés par l'intermédiaire de leurs motifs photoactivables- D'où il s'ensuit que l'invention concerne des polymères, monomères et oligomères caractérisés en ce qu'ils comprennent des substituants photogreffés obtenus par réaction d'une ou plusieurs substances avec leurs motifs photoactivables, ces substances étant de préférence choisies parmi les (macro)molécules biologiques et/ou les chimères de celles-ci et/ou parmi les unités de ces (macro)molécules et/ou de ces chimères.

A titre d'exemple de substances photogreffées on peut citer les acides aminés, les oligo peptides, les polypeptides, les protéines, les gluco-protéines, les lipoprotéines ou bien encore les nucléotides et oligonucléotides, les polynucléotides type ARN ou ADN, d'origine biologique ou synthétique, des enzymes, des coenzymes et des vitamines.

Comme exemples de chimères de biomolécules on peut citer les (poly)Acides Nucléiques Peptidiques, des sites prosthétiques d'enzymes tels que des métalloporphyrines, des isobactériochlorines, des corrines, des chlorins, des anticorps artificiels à base par exemple de polymère à empreinte.

Selon d'autres aspects avantageux de l'invention, l'invention couvre encore :

- des polymères, ou monomères, caractérisés en ce que les (macro)molécules biologiques photogreffés précitées sont choisies parmi le groupe consistant des acides aminés, des oligopeptides, des polypeptides, des protéines, des gluco-protéines, des lipoprotéines, des nucléotides, des oligonucléotides, des polynucléotides type ARN ou ADN, d'origine biologique ou synthétique, des enzymes, des co-enzymes et des vitamines ; les chimères de biomolécules précitées sont choisies parmi le groupe consistant des (poly)acides nucléiques peptidiques, des sites prosthétiques d'enzymes tels que des métalloporphyrines, des isobactérioclorines, des corrines, des chlorins, des anticorps artificiels à base par exemple de polymères à empreinte,
- un monomère pyrrolbenzophénone de formule :

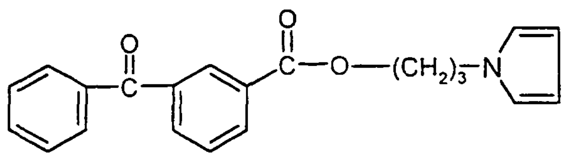

(benzophénone) (pyrrole)

en particulier obtenu par réaction de l'acide 3-benzoylbenzoïque avec le 3-pyrrol-1-propanol,

- un monomère 4-(2-aminoéthyl)phénol-benzophénone de formule :

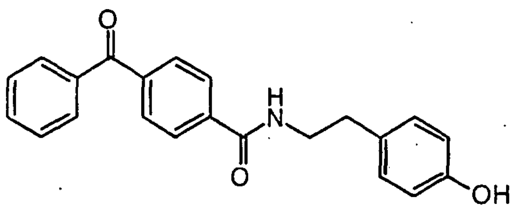

en particulier obtenu par réaction du 4-(2-aminoéthyl)phénol avec l'acide 4-benzoylbenzoïque,

- un monomère indol-benzophénone de formule :

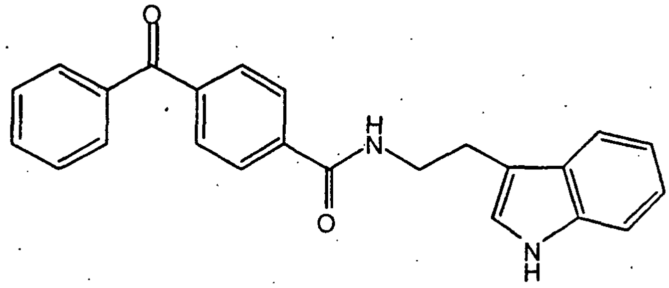

en particulier obtenu par réaction du 3-(2-aminoéthyl)indole avec l'acide 4-benzoylbenzoïque,

- un monomère vinylaniline-benzophénorie de formule :

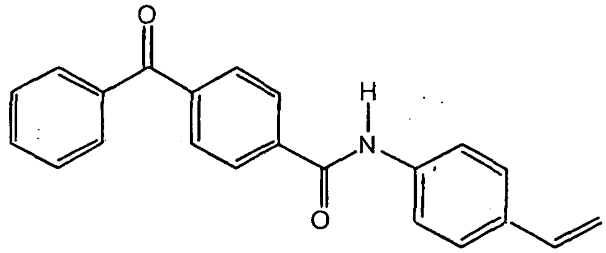

en particulier obtenu par réaction de la vinylaniline avec l'acide 4-benzoylbenzoïque,

- un monomère azidophénylpyrrole de formule :

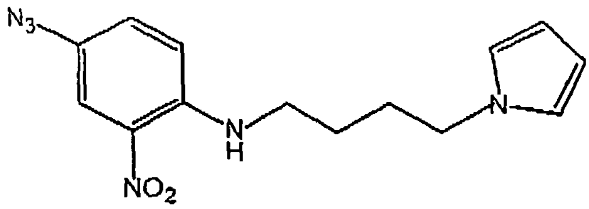

en particulier obtenu par réaction de 4-fluoro-3-nitro-phénylazide avec l'aminobutylpyrrole,

- un électropolymère photogreffable, caractérisé en ce qu'il est préparé à partir d'un monomère, selon l'invention.
- un poly(pyrrolbenzophénone) obtenu à partir du pyrrole-benzophénone précité, de préférence photogreffé avec une substance photogreffable telle que définie dans la description et les revendications, encore mieux choisie parmi une protéine, de préférence l'albumine de sérum bovin ou humain, une enzyme, de préférence la glucose oxydase ; une biotine, de préférence le système affin avidine-biotine, la thionine ; un anticorps, de préférence un anticorps de lapin (IgG),
- un polymère de polyphénol-benzophénone obtenu à partir du monomère phénol-benzophénone précité, de préférence photogreffé par une substance photogreffable telle que définie dans la description et les revendications, encore mieux choisie parmi une enzyme, de préférence la glucose oxydase,
- un polymère poly-indol-benzophénone obtenu à partir du monomère indol-benzophénone précité, de préférence photogreffé par une substance photogreffable telle que définie dans la description et les revendications,
- un polymère de polyvinyl-benzophénone obtenu à partir du monomère vinylaniline-benzophénone précité, de préférence comportant une substance photogreffable telle que définie dans la description et les revendications,
- un polymère de polypyrrol-phénylazide obtenu à partir de la photopolymérisation du phénylazide-pyrrole précité, de préférence photogreffée par une substance photogreffable telle que définie dans la description et les revendications, encore de préférence une enzyme en particulier la glucose oxydase.

Selon encore un autre de ses aspects, la présente invention concerne un procédé de fabrication de biocapteurs électroniques du type de ceux comprenant au moins un (micro)transducteur comportant au moins une membrane

sensible porteuse de sondes de reconnaissance spécifiques de molécules cibles à analyser, caractérisé en ce que l'on réalise la membrane sensible fixée sur le support transducteur à partir d'au moins un polymère tel que décrit supra et en ce que l'on greffe (de préférence de manière localisée), les sondes de reconnaissance spécifique sur les motifs photoactivables du polymère par photoactivation.

Ces biocapteurs ou ces éléments de biocapteurs peuvent être des (micro)électrodes, des dispositifs semi-conducteurs tels que des transistors effet de champ ou bien encore des réseaux de microélectrodes interdigitées, des quartz de microbalance, des fibres de carbone ou de graphite micrométrique et nanométriques, des fibres optiques recouvertes d'un film conducteur.

Ces (micro)conducteurs sont aptes à servir de support à la croissance des électropolymères photogreffables selon l'invention. Dès lors que l'électropolymère est formé sur le microtransducteur dans les endroits souhaités, on met en oeuvre le photogreffage. A cette fin, on irradie le film électropolymère e. g. à l'aide d'une lampe par exemple à 300-400 nm (selon le motif photoactivable considéré), en présence de molécules ou biomolécules à greffer (sondes de reconnaissance spécifique). Ces molécules ou biomolécules à greffer sont mises en oeuvre sous forme de suspension, dispersion ou en solution dans un milieu organique ou aqueux, d'émulsion ou solubilisées dans des micelles ou des micelles inverses.

Les dispositifs d'irradiation sont des moyens connus en eux-mêmes. Il est ainsi parfaitement envisageable de réaliser l'irradiation en continu dans un tunnel à l'échelle industrielle.

La présente invention concerne encore un biocapteur électronique du type de ceux comprenant au moins un (micro)transducteur comportant au moins une membrane sensible porteuse de sondes de reconnaissance spécifique caractérisé en ce que la membrane sensible comprend au moins un polymère tel que décrit supra, sur lequel sont photogreffés (de préférence de manière localisée), des substituants qui correspondent aux sondes de reconnaissance spécifique.

Les microtransducteurs sur lesquels peuvent être immobilisées des biomolécules grâce aux électropolymères selon l'invention, peuvent être des microélectrodes, des microélectrodes interdigitées, des transistors à effet de champ, des biopuces notamment pour le séquençage de l'ADN et de l'ARN ou des protéines.

Ces biopuces peuvent se présenter sous forme de réseaux matriciels de sondes de reconnaissance spécifique constituées par exemple par des oligonucléotides fixés de manière localisée sur des structures isolant/semi-conducteur, fonctionnant sur le principe de l'effet de champ, par exemple.

La technologie selon l'invention est particulièrement adaptée à la fabrication de ce type de capteurs grâce à ces capacités de contrôle spatial de la fixation des sondes par adressage électrochimique et par sa simplicité de greffage des molécules par photoactivation et photomasquage.

A titre d'autres exemples de biocapteurs on peut citer les immunocapteurs à lecture optique ou électrique mais également les microbalances à quartz.

L'invention concerne enfin un support formant électrode, caractérisé en ce qu'il comprend à sa surface au moins une couche d'un polymère tel que défini dans la description et les revendications.

La présente invention sera mieux comprise à la lumière des exemples qui suivent et qui décrivent l'obtention d'électropolymères photogreffables, leur caractérisation, le greffage de protéines sur ces électropolymères et la caractérisation des produits greffés obtenus. Ces exemples permettent également de faire ressortir tous les avantages et les variantes de l'invention.

## EXEMPLES

### Description des figures

**La Fig. 1** représente le spectre obtenu par spectroscopie infrarouge (IR) du monomère pyrrole-benzophénone sous sa forme liquide tracé en mode transmission.

**La Fig. 2** est une courbe donnant la caractérisation électrochimique du monomère obtenu à l'exemple 1.1 à savoir le pyrrole-benzophénone.

**La Fig. 3** est un voltamogrammé illustrant l'électropolymérisation du monomère de l'exemple 1.1 pyrrole-benzophénone.

**La Fig. 4** est une courbe de voltamétrie cyclique obtenue sur l'électrode revêtue de l'électropolymère photogreffable poly(pyrrole-benzophénone) obtenu à l'exemple 2.

**La Fig. 5** montre 2 courbes représentant la variation de la fréquence F (Hz) en fonction du temps t (Δ), cette variation résultant de l'immobilisation d'avidine (exemple 3.2) sur des quartz de microbalance modifiés par des films de poly (pyrrole-benzophénone) et préalablement mis en contact avec une solution aqueuse de biotine avec irradiation (----) et sans irradiation (----).

**La Fig. 5** montre 2 courbes représentant la variation de la fréquence F (Hz) en fonction du temps t (Δ), cette variation résultant de l'immobilisation d'avidine (exemple 3.2) sur des quartz de microbalance modifiés par des films de poly (pyrrole-benzophénone) et préalablement mis en contact avec une solution aqueuse de biotine avec irradiation (----) et sans irradiation (----).

La **Fig. 6** représente le spectre infrarouge du film polymère polypyrrole-benzophénone obtenu à partir du monomère dont le spectre IR est représenté à la figure 1.

La **Fig. 7** représente en courbe A le signal électrochimique du film polymère polypyrrole-benzophénone dont le spectre infrarouge est montré à la Fig. 6.

La **Fig. 8** représente en courbe B le signal électrochimique enregistré avec une électrode nue par cyclage en potentiel dans une solution de thionine à 0,5 mM dans l'acétonitrile ; tandis que le signal électrochimique du film de polymère de polypyrrole-benzophénone après photogreffage de la thionine comme espèce électroactive est représenté en courbe C de la **Fig. 8**.

Les **Fig. 9 et 10** représentent respectivement : la **Fig. 9** en courbe A le signal électrochimique du film de polypyrrole-benzophénone, sans excitation lumineuse, avant trempage dans la solution de thionine à 0,5 mM dans l'acétonitrile, tandis que la courbe après trempage ou immersion dans cette solution est représentée par la courbe B de la **Fig. 10.**

Les **Fig. 11 et 12** représentent respectivement les courbes d'intensité (I) en ordonnées en microampère obtenu par balayage de potentiel en système rédox $Ag/Ag^+$ en abscisse du film de polypyrrole-benzophénone avant trempage ou immersion dans une solution **(Fig. 11)** dans une solution d'anticorps de lapin, ou après immersion et irradiation **(Fig. 12).**

Les **Fig. 13 et 14** sont des courbes similaires à celles des **Fig. 11 et 12** respectivement, sans excitation lumineuse.

La **Fig. 15** représente les courbes de Koutecaky-Levich dans lesquelles la droite reliant les carrés est celle qui a été obtenue avec le tracé I(E) de la **Fig. 11** et la courbe reliant les cercles est celle obtenue avec la courbe I(E) de la **Fig. 12** après trempage et excitation lumineuse (hω).

La **Fig. 16** est une courbe similaire à celle de la figure 15 mais obtenue avec les tracés I(E) respectivement des **Fig, 13 et 14**, sans excitation lumineuse.

Les **Fig. 17 et 18** représentent respectivement les signaux électrochimiques du film polypyrrone-benzophénone avant trempage (**Fig. 17**) et après trempage et irradiation (**Fig. 18**) dans une solution d'anticorps de lapin (IgG de lapin).

Les **Fig. 19 et 20** représentent respectivement des courbes avant trempage (**Fig. 19**) et après trempage sans irradiation (**Fig. 20**) dans les mêmes conditions que celles des **Fig. 17 et 18**, à titre comparatif afin de montrer que la modification du signal électrochimique DEm en solution est moindre pour une électrode non irradiée.

La **Fig.21** représente un spectre RMN du monomère 4-(2-aminoéthyl)phénol-benzophénone.

La **Fig. 22** représente en courbe A le signal électrochimique du monomère de l'exemple 4 et ayant un spectre RMN de la **Fig. 21** lors de balayages successifs de potentiel entre 0 et 1 Volt dans un système rédox Ag-AgCl et en courbe B le signal obtenu par cyclage en réduction dans la même solution de monomère.

La **Fig. 23** représente la courbe intensité/potentielle obtenue après des balayages répétitifs de potentiel entre 0 et 1 volt d'une électrode transférée dans une solution électrolytique exempte de monomère cyclé dans le domaine d'électroactivité de la benzophénone.

La **Fig. 24** représente la courbe d'absorbance en fonction du temps, à 425 nm, de l'activité enzymatique d'une électrode revêtue d'un film de polypyrrole-benzophénone sur lequel a été photogreffé l'enzyme glucose oxydase, selon l'exemple 5.1 ci-après, afin de montrer la conservation de l'activité enzymatique de l'éléctrode modifiée.

La **Fig.25** représente le spectre RMN du monomère 3-(2-aminoéthyl)indol-benzophénone des exemples 6.1 et 6.2.

La **Fig. 26** représente le spectre RMN du monomère vinylaniline-benzophénone des exemples 7.1 et 7.2.

La **Fig. 27** représente le spectre RMN du monomère 4-fluoro-3-nitro-phénylazide-aminobutylpyrrole des exemples 8-1 et 8-2.

La **Fig. 28** représente le voltammogramme de polymérisation du monomère objet du spectre RMN de la **Fig. 27** montrant dans la partie anodique un pic irréversible à +0,95 $V/Ag/Ag^+$ correspondant à l'oxydation du groupe pyrrole.

La **Fig. 29** représente en courbe A le signe de l'électrode immergé dans la solution de monomère 4-fluoro-3-nitro-phénylazide-aminobutylpyrrole synthétisé à l'exemple 8-2 polymérisé par balayage répétitif de potentiel entre 0 et 0,90 $V/Ag/Ag^+$, montrant la formation de polymère par l'apparition et la croissance d'un système rédox à + 0,4 $V/Ag/Ag^+$ caractéristique de l'électroactivité des chaînes polypyrroliques.

La **Fig. 30** représente en courbe B le signal de l'électrode objet de l'expérience de la **Fig. 29** après une électrooxydation de 2 mC et son transfert dans une solution électrolytique exempte de monomère.

La **Fig. 31** représente une courbe d'étalonnage du glucose en milieu aqueux avec une électrode revêtue d'un film de polymère de poly[aminobutylpyrrole] comportant des motifs 4-fluoro-3-nitro-phénylazide obtenus par polymérisation du monomère de l'exemple 8-2, dans les conditions de l'exemple 8-3 et sur lequel a été greffé l'enzyme glucose oxydase dans les conditions de l'exemple 8-4, en milieu aqueux à + 600 $mV/Ag/Ag^+$ afin d'oxyder à la surface de l'électrode les molécules de $H_2O_2$ enzymatiquement générées comme indiqué dans le cadre de l'exemple

8-4.
La **Fig. 32** représente une courbe de Lineweaver-Burk de calibration avec en ordonnées l'inverse des intensités exprimées en nanoampère$^{-1}$, et en abscisse l'inverse de la concentration en glucose exprimée en millimole$^{-1}$, permettant d'accéder à la constante apparente de Michaelis Menten, ici obtenue égale à 20 mM, similaire à celle de la constante cinétique de l'enzyme libre glucose oxydase en solution.

## PARTIE I - PREPARATION DE MONOMERES ET DE POLYMERES CONJUGUES A DES MOTIFS PHOTOACTIVABLES DE BENZOPHENONE

### 1.1 - Monomère pyrrole benzophénone

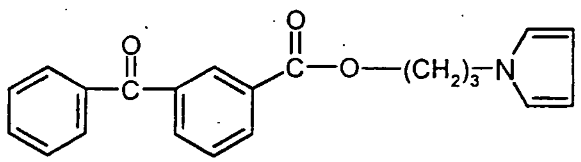

(benzophénone) (pyrrole)

Ce monomère est préparé comme indiqué ci-après :

Réaction du pyrrole alcool avec de l'acide 3-benzoyl-benzoïque en présence de DMAP.

Selon un mode de réalisation actuellement préféré, on mettra à réagir de l'acide 3-benzoylbenzoïque avec le 3-pyrrole-1-propanol en présence d'une quantité catalytique de diméthylaminopyrridine ou DMAP et de préférence en présence d'un agent activateur tel que la dicyclohexylcarbodiimine.

La réaction de synthèse est en pratique la suivante:

425 mg d'acide 3-benzoylbenzoïque (2 mmoles) et 413 mg de dicyclohexylcarbodiimine (2 mmoles) sont dissous dans 20 ml de dichlorométhane et laissés sous agitation durant 5 min. On ajoute ensuite 208 mg de 3-pyrrole-1-propanol (1,66 mmole) au mélange réactionnel et on laisse réagir pendant 5 min sous agitation. On ajoute ensuite une quantité catalytique de diméthylaminopyrridine, ou DMAP (81 mg, 0,66 mmole). On maintient le mélange réactionnel à 20°C environ sous agitation pendant 48 heures.

Après ajout d'eau et extraction au dichlorométhane, la solution organique est évaporée sous pression réduite.

Le résidu est alors dissous dans l'éther éthylique et la solution obtenue est filtrée afin d'éliminer les produits insolubles. Après évaporation du solvant, le produit est chromatographié sur une colonne de silice avec comme éluant le dichlorométhane.

Le dérivé pyrrolique de la benzophénone est alors obtenu sous forme d'une huile quasi-limpide avec un rendement d'environ 61 %.

Après chromatographie, la pureté du pyrrole-benzophénone a été confirmée par RMN et spectre de masse. H RMN (CD $Cl_3$) S (ppm) 2,20 (m, 2H), 4,1 (t, 2H), 4,29 (t, 2H), 6, 10 (t, 2H), 6,60 (t, 2H), 7,52 (m, 4H), 7,80 (d, 2H), 7,98 (d, 1H), 8,22 (d, 1H), 8,40 (s, 1H).

Spectrométrie de masse par impact électronique : 333 (100 %), 209 (14 %), 152 (11 %), 124 (21 %).

On a tracé le spectre par spectroscopie infrarouge (IR) du monomère pyrrole benzophénone sous sa forme liquide à l'aide d'un dispositif de réflexion spéculaire, en mode transmission, qui est représenté à la figure 1, ici le dispositif de réflexion spéculaire disponible dans le commerce sous le nom commercial Perkin-Elmer Spectrum GX FT IR System capable de fonctionner soit en mode transmission, soit en mode réflexion spéculaire.

Les propriétés électrochimiques de ce produit ont été analysées par voltamétrie cyclique.

Comme l'indique la **Fig. 2** annexée ci-dessus, le pyrrole benzophénone s'oxyde irréversiblement à 1,06 V vs $Ag/Ag^+$ 10 mM dans $CH_3CN$. L'oxydation irréversible de ce monomère consiste en là formation d'un pyrrole radical cation, 1ère étape d'un phénomène de polymérisation conduisant au polypyrrole.

Ces radicaux pyrroliques se couplent entre eux donnant des liaisons covalentes et libérant des protons dans le milieu électrolytique. Cette libération de protons est illustrée lors du balayage retour par l'apparition d'un pic cathodique irréversible à environ 200 mV (**Fig. 2**).Ce pic correspond à la réduction, à la surface de l'electrode de platine, des protons libérés lors du phénomène de polymérisation des pyrrole-benzophénone. Après couplage de deux radicaux pyrroliques et perte de deux protons conduisant à la réaromatisation du dimère, ce dernier peut réagir de manière équivalente avec un radical cation du pyrrole-benzophénone. Par propagation électrochimique continue, une chaine polypyrrolique est

ainsi générée conduisant à un polymère insoluble. En milieu aprotique, le radical anion de la benzophénone étant stable, la benzophénone peut se réduire réversiblement à 1 électron. Effectivement, dans le domaine cathodique, un signal réversible à - 1,96 V (**Fig. 2**) correspondant à la réduction monoélectronique du pyrrole-benzophénone est observé.

## EXEMPLE 2 - ELECTROPOLYMERISATION

### 2.1 -Obtention poly(pyrrole benzophénone)

Des balayages répétitifs de potentiel entre 0 et 0,86 V entraînent la formation d'un film pyrrolique à la surface de l'électrode. Cette formation est illustrée par l'apparition et la croissance d'un système rédox à + 0,32 V caractéristique de l'électroactivité des chaînes polypyrroliques (cf **Fig. 3** Electropolymérisation du pyrrole benzophénone 3,6 mM dans $CH_3CN$ + 0,1 M TBAP).

La formation du film de poly(pyrrole benzophénone) à la surface de l'électrode est avantageusement réalisée à potentiel constant égal à 900 mV/Ag/$Ag^+$.

Après transfert de l'électrode dans une solution d'électrolyte exempte de monomère, le voltamogramme de cette électrode présente l'électroactivité classique des films polypyrroliques dans le domaine anodique. De plus, le signal réversible de la réduction des motifs benzophénone à $E_{1/2}$ = - 1,96 V confirme la présence d'un film polymère à la surface de l'électrode (cf. **Fig. 4** : Voltamétrie cyclique de l'électrode après polymérisation) ainsi que la présence des motifs photoactivables dans ce polymère.

L'intégration de la charge relative à l'oxydation des chaines polypyrroliques permet de calculer la quantité de monomères polymérisés à la surface de l'électrode. En opérant de cette manière, on obtient un film de poly(pyrrole benzophénone) correspondant à 1,8 $10^{-8}$ mole.$cm^{-2}$.

### 2.2 -Spectroscopie infrarouge démontrant l'obtention d'un film de poly(pyrrole benzophénone)

Les films de poly(pyrrole benzophénone) obtenus à l'étape 2.1 ci-dessus par électrochimie sont caractérisés en spectroscopie infrarouge (IR), à l'aide du dispositif de réflexion spéculaire précité. Cette méthode est adaptée à l'analyse de revêtements de surface car l'importance de la lumière réfléchie dépend de la rugosité de surface et des propriétés d'absorption de l'échantillon.

Il est rappelé qu'afin d'observer les changements entraînés par la polymérisation, le spectre IR du monomère sous sa forme liquide a été préalablement tracé en mode transmission et représenté en figure 1.

Le spectre infrarouge du film polymère poly(pyrrole benzophénone) est représenté en figure 6, en mode réflexion spéculaire.

On observe à partir d'une comparaison des figures 1 et 6 que du fait de l'utilisation de deux méthodes différentes, à savoir respectivement en transmission en figure 1 et réflexion spéculaire en figure 6, et du changement d'état du produit, respectivement liquide et film, le spectre de la figure 6 est beaucoup moins chargé et l'intensité des raies obtenues très diminuée. La totalité des pics observés sont présents sur le spectre du monomère. Ce spectre obtenu de façon reproductible montre que le film une fois formé conserve les groupements fonctionnels nécessaires au greffage par photoactivation.

## EXEMPLE 3 - PHOTOGREFFAGE DE BIOMOLECULES SUR LES ELECTROPOLYMERES PHOTOGREFFABLES DE L'EXEMPLE 2

### Mise en évidence du photogreffage de différentes espèces aminées

Le groupe fonctionnel benzophénone est connu pour être le précurseur de la photogénération de biradicaux cétone. Ces biradicaux sont accepteurs d'hydrogène et l'arrachement d'un proton des molécules cibles précède la formation d'une liaison covalente. Cette photolyse réalisée à 350 nanomètres (nm) a été largement décrite en solution.

Selon la présente invention, l'efficacité du photogreffage sur les films de poly(pyrrole benzophénone) a été mise en évidence par électrochimie.

### 3.1. - Greffage d'une espèce électroactive : la thionine

Des embouts de carbone vitreux, recouverts d'un film de polypyrrole benzophénone (PP BP) formé par le passage de 0,2 mC (millicoulomb) ont été utilisés. Ces embouts ont été immergés et irradiés pendant deux heures par une lampe à mercure de 250 W à travers un filtre IR avec une intensité de 100mWcm-2. L'irradiation de lumière entre 300 et 350 nm est réalisée dans une solution organique de thionine 1 mM, désoxygénée au préalable par bullage d'argon durant 30 min.

Le schéma réactionnel de principe est donné ci-après :

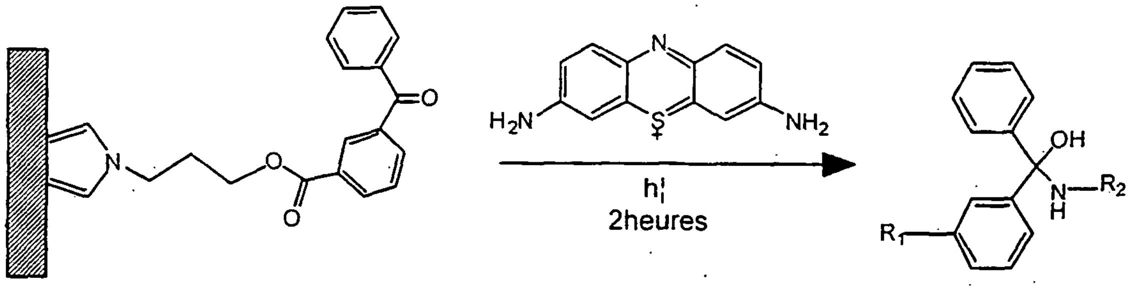


Le signal électrochimique des électrodes dans l'acétonitrile a été enregistré avant et après greffage. La figure 7 représente en courbe A le signal électrochimique du film avant greffage de l'espèce électroactive. La figure 8 représente en courbe B le signal enregistré avec un embout nu par cyclage en potentiel dans une solution de thionine à 0,5 mM dans l'acétonitrile. La courbe C de la figure 8 représente le signal électrochimique du film après photogreffage de l'espèce électroactive.

L'apparition du signal quasi réversible de la réduction de la thionine sur le voltampérogramme de l'électrode modifiée démontre la présence de l'espèce dans le volume du film. En effet, la réduction électrochimique de la thionine ne peut intervenir qu'à la surface de l'électrode et ensuite se propage par saut d'électron entre sites thionines immobilisés.

La même expérience réalisée sans excitation lumineuse des embouts immergés (blanc) n'entraîne pas de modification significative du signal électrochimique du film de polymère.

La courbe A obtenue avant trempage est représentée à la figure 9 et la courbe B obtenue après trempage ou immersion est représentée à la figure 10.

Le signal réversible de la réduction des motifs benzophénone a légèrement diminué en raison probablement d'une photodégradation, par contre le signal de la thionine n'apparaît pas. Ceci démontre clairement la nécessité d'une photoactivation du film pour obtenir un greffage chimique des sondes électroactives.

Le taux moyen de greffage de motifs thionine est évalué à 18 % par comparaison via l'intégration des charges électriques , de la quantité de thionine immobilisée par rapport à la quantité de benzophénone polymérisée.

**3.2** - **Poly(pyrrole benzophénone) - anticorps de lapin (IgG)**

**3.2. A - Démonstration de la photofixation d'anticorps de lapin (IgG) sur un film de polypyrrole-benzophénone ou PPBP, par des mesures de perméabilité**

La détermination de la perméabilité de films et de la cinétique de transfert de charge d'un système rédox est basée sur le tracé des courbes intensité/potentiel (I(E)) en régime stationnaire, d'oxydation ou de réduction à la surface d'une électrode d'une espèce électroactive. Les mesures sont effectuées sur embout d'électrodes tournantes, à faible vitesse de balayage en tension.

Les mesures de perméabilité d'un film sont basées sur la détermination de la vitesse à laquelle le soluté électroactif diffuse à travers le film pour être oxydé ou réduit à la surface de l'électrode. Ces mesures sont donc réalisées à l'aide de sondes électroactives susceptibles de traverser le film ayant une taille adéquate. Si le film est conducteur, il est nécessaire soit de travailler dans un domaine de potentiel où le polymère n'est pas conducteur, soit de détruire l'électroactivité du film afin d'éviter des interférences. En effet, le film peut jouer le rôle de médiateur rédox pour l'oxydation ou la réduction des sondes électroactives. Cet aspect conditionne en partie le choix des sondes : le potentiel rédox des espèces doit se trouver dans un domaine pour lequel le film n'est pas conducteur. Le deuxième critère est la taille des sondes : les mesures de perméabilité sont souvent réalisées avec plusieurs sondes dont l'encombrement stérique diffère.

Dans le cadre de la présente invention, les inventeurs ont choisi de travailler avec le décaméthylferrocène (DMFc), espèce électroactive dont l'oxydation réversible a lieu en milieu organique à -100 mV/Ag/$Ag^+$.

Les courbes d'intensité en fonction du potentiel (I(E)) ont été obtenues avec des embouts d'électrodes tournantes en carbone vitreux recouverts d'un film de PPBP (0,5 mC (millicoulomb), avant et après immersion dans une solution aqueuse désoxygénée d'anticorps de lapin (IgG) à une concentration de 0,5 $mgml^{-1}$, et irradiée pendant 2 heures, à l'aide d'une source de lumière issue d'une lampe à mercure de 250 W à travers un filtre IR avec une intensité de 100mWcm-2

Une fois rincées, les électrodes sont plongées dans une solution de DMFc, 0,5 mM dans l'acétonitrile et le courant d'oxydation de la sonde électrochimique est enregistré en fonction du potentiel pour différentes vitesses de rotation des électrodes.

Les tracés obtenus sont représentés respectivement aux figures 11 (avant trempage) et 12 (après trempage).

On remarque à partir de la considération des figures 11 et 12 qu'après trempage et irradiation, les courants limites de diffusion ont diminué et que le potentiel d'oxydation de la sonde est décalé vers des valeurs plus élevées. Ces deux phénomènes peuvent être attribués à la présence d'anticorps greffés en surface, modifiant la mobilité de la sonde dans le film résultant et la cinétique du transfert de charge du processus électrochimique.

La même expérience réalisée sans excitation lumineuse des embouts immergés (trempage) n'entraîne pas de modification significative du courant d'oxydation de la sonde électrochimique comme montré aux figures 13 et 14 respectivement avant trempage et après trempage ou immersion. Ceci démontre qu'en l'absence d'irradiation lumineuse, les anticorps ne sont pas fixés.

En ce qui concerne les courbes I(E) des figures 11 à 14, il est rappelé que la détermination de la perméabilité est réalisée à l'aide des courbes de Koutecky - Levich basées sur l'équation suivante :

$$\frac{C_s^*}{i_{lim}} = \frac{\delta_m}{KD_m nFA} + \frac{1}{0,62 D_s^{2/3} \gamma^{1/6} nFA} * \frac{1}{\omega^{1/2}}$$

- $C_s^*$ concentration de l'espèce électroactive en solution
- $\omega$ vitesse de rotation de l'électrode tournante
- $D_s$ coefficient de diffusion de l'espèce en solution
- $D_m$ coefficient de diffusion de l'espèce dans le film
- $\delta_m$ épaisseur du film
- $i_{lim}$ courant limite de diffusion

la perméabilité correspond au rapport suivant :

$$P_m = \frac{KD_m}{\delta_m}$$

- $\gamma$ viscosité cinématique de la solution
- n nombre d'électrons échangés lors du processus faradique
- F faraday
- A surface de l'électrode
- K coefficient de partage de l'espèce entre le film et la solution

Les courbes représentent la variation du rapport $\frac{C_s^*}{i_{lim}}$ en fonction de $\frac{1}{w^{1/2}}$. Connaissant n, F et A, l'ordonnée à l'origine permet d'obtenir la valeur de la perméabilité $P_m$.

$i_{lim}$ est obtenu en traçant les courbes intensité/potentiel en régime stationnaire, pour différentes vitesses de rotation d'électrode.

L'exploitation des tracés I(E) précédents a permis d'obtenir les courbes de Koutechy-Levich représentées à la figure 15 où l'ordonnée à l'origine est inversement proportionnelle à la perméabilité des films pour la sonde.

On remarque de la figure 15, qu'après greffage, la perméabilité du film a diminué. Les mêmes courbes obtenues avec les tracés I(E) d'une électrode non irradiée (figure 16) convergent vers la même ordonnée à l'origine, ce qui indique que la perméabilité du film n'a pas été modifiée par une simple immersion ou trempage des électrodes dans la solution d'anticorps.

### 3.3 - Poly(pyrrole benzophénone) - ASB

L'irradiation d'un film de poly (p-pyrrole benzophénone) entraîne, en voltamétrie cyclique, une forte diminution (-54%) de l'intensité du sytème rédox à -2 V relatif à la fonction benzophénone. ce phénomène illustre la disparition de la fonction cétone et donc la réactivité de ce groupement sous irradiation.

Les macromolécules biologiques étant solubles quasi exclusivement en milieu aqueux, l'efficacité du greffage par irradiation a été testée en milieu aqueux. Dans ce but, on utilise dans un premier temps l'albumine de sérum bovin (ASB) comme protéine modèle. Des électrodes modifiées ont été plongées dans une solution aqueuse de cette protéine et irradiées avec une lumière issue d'une lampe à mercure de 250 W à travers un filtre IR avec une intensité de 100mWcm-2

Afin de détecter l'immobilisation de protéines à la surface du film, ces électrodes ont été transférées dans un électrolyte organique et la perméabilité du film a été testée à l'aide d'une sonde électroactive (ferrocène). Une diminution de 25% vis-à-vis de la perméabilité initiale apparaît confirmant le greffage de protéine. Il faut également remarquer que cette diminution n'est pas due à une simple adsorption de la protéine à la surface du film. En effet, la même expérience réalisée sans irradiation n'entraîne qu'une perte de 9 % de la perméabilité du film.

### 3.4 - Poly(pyrrole benzophénone) - enzyme glucose oxydase

Pour confirmer ces possibilités de greffage, l'irradiation des électrodes, modifiées dans les conditions de l'exemple 3.3, a été réalisée en présence d'une autre protéine biologiquement active : une enzyme. Cette enzyme, la glucose oxydase ou GOD, catalyse l'oxydation du glucose en acide gluconique en présence de dioxygène qui lui est réduit en $H_2O_2$.

Comme $H_2O_2$ s'oxyde électrochimiquement au potentiel de 0,6 V versus ECS sur une électrode de platine, cette réaction enzymatique peut être suivie par un signal électrique. Par conséquent, après irradiation, l'electrode modifiée est transférée dans une solution aqueuse et maintenue au potentiel de 0,6 V vs ECS. En présence de glucose, l'enzyme forme $H_2O_2$ qui, à ce potentiel, est oxydé à la surface de l'electrode de platine, générant ainsi un courant électrique.

La réponse ampérométrique de l'électrode à des ajouts de glucose démontre tout d'abord la possibilité de photogreffer une enzyme sur ce type de polymère tout en conservant son activité biocatalytique vis à vis de ses subsrats. Cette réponse permet également d'établir une courbe de calibration du glucose dont la pente de la partie linéaire I (intensité = f (concentration en glucose) indique la sensibilité du capteur : 3,37 $mAM^{-1}$ $cm^{-2}$.

La même expérience réalisée sans irradiation démontre que l'enzyme peut s'adsorber sur le film polymère mais la sensibilité au glucose obtenue (0,56 $mAM^{-1}$ $cm^{-2}$) est nettement inférieure à la précédente. Ceci confirme le greffage de l'enzyme sous irradiation du film de poly (pyrrole benzophénone).

### 3.5 - Poly (pyrrole benzophénone) - biotine

L'efficacité du photogreffage et l'accessibilité de la biomolécule immobilisée à la surface du polymère ont été examinées par des mesures gravimétriques avec le système affin avidine-biotine.

Des électrodes d'or recouvrant des quartz de microbalance ont été modifiées par un film de poly (pyrrole benzophénone) correspondant à 2 $10^{-9}$ $molecm^{-2}$. Deux quartz modifiés ont été mis en contact avec une solution aqueuse de biotine, l'un étant irradié et l'autre pas. En raison des fortes interactions affines entre la biotine (une vitamine, PM 250) et l'avidine (une protéine, PM 66000), l'association avidine-biotine s'effectue spontanément. Les mesures gravimétriques, en présence d'une solution d'avidine (0,5 mg/ml), indiquent une diminution de fréquence de 200 Hz et 70 Hz pour respectivement le polymère irradié et le polymère non irradié (voir **Figure 4**). Ceci correspond à un accroissement de masse à la surface du polymère de 358 ng $cm^{-2}$ dans le cas de l'irradiation et de 125 ng $cm^{-2}$ en absence d'irradiation.

Or l'immobilisation d'une monocouche compacte d'avidine correspond théoriquement à un accroissement de masse de 362 ng $cm^{-2}$. Il apparaît donc que l'irradiation induit le greffage efficace de biotine à la surface du polymère entrainant un recouvrement total de ce dernier par une monocouche d'avidine. En l'absence d'irradiation et donc de photogreffage de biotine, uniquement 34 % de la surface du polymère est recouverte d'avidine.

Ces résultats démontrent également que la biotine greffée conserve ses propriétés de reconnaissance vis à vis de l'avidine et conserve donc une excellente accessibilité. Les mesures gravimétriques ont démontré qu'une monocouche d'avidine était immobilisée à l'interface polymère-solution soit 5,48 $10^{-12}$ $mole.cm^{-2}$. Par conséquent au minimun 5,48 $10^{-12}$ $mole.cm^{-2}$ de biotine sont photogreffées à la surface du polymére. Sachant qu'une monocouché de polymère peut être approximée à 8 $10^{-11}$ $mole.cm^{-2}$, ceci conduit à un taux de greffage minimun de 6 %.

**3.2-B- Démonstration à la photofixation d'anticorps sur un film de poly(pyrrole-banzophénone)-PPBP par voltammétrie cyclique.**

Des embouts de carbone vitreux recouverts d'un film de PPBP (0,5 mC) sont immergés dans une solution d'anticorps de lapin (IgG) désoxygénée au préalable par bullage d'argon durant 30 min et irradiés avec l'aide de la même lumière issue d'une lampe à mercure de 250 W, pendant deux heures.

Les voltammétries cycliques des Em dans une solution de DMFc (0,5 mM) ont été enregistrées avant et après immersion ou trempage.

Les courbes obtenues font l'objet des figures 17 et 18 respectivement.

On peut observer à partir des figures 17 et 18 que les voltamogrammes présentent dans le domaine des potentiels positifs l'électroactivité classique des films polypyrroliques et dans le domaine des potentiels négatifs l'oxydation irréversible du DMFc.

Ce signal disparaît complètement après greffage du film par les anticorps IgG de lapin.

La modification du signal électrochimique des Em dans la solution de DMFc est moindre pour une électrode non irradiée, comme montré par les courbes obtenues sans irradiation faisant l'objet des figures 19 et 20.

On observe que le courant d'oxydation de la sonde a légèrement diminué alors qu'il disparaît complètement pour une électrode irradiée.

L'ensemble des résultats obtenus tend à prouver que l'immobilisation de différentes molécules est possible à la surface des films de PPBP. Cette immobilisation nécessite cependant une excitation lumineuse des films.

Un simple trempage n'entraîne pas de modification.

Ce produit n'a donc pas d'interaction de type adsorption, mais la formation photoactivée de liaisons covalentes.

**PARTIE II - PREPARATION DE DIFFERENTS MONOMERES ELECTROPOLYMERISABLES FONCTIONNALISES PAR DES MOTIFS PHOTOACTIVABLES DE BENZOPHENONE.**

**Exemple 4 - Monomères de type phénol-benzophénone, ici par exemple de 4-(2-aminoethyl)phénol(tyramine)-benzophénone.**

La formule de ce monomère nouvellement synthétisé est la suivante :

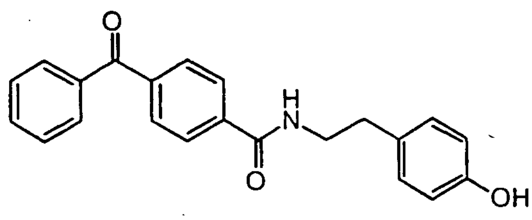

**4.1-Synthèse de ce nouveau dérivé**

Dans un ballon de 50ml mis sous argon, on introduit 452mg d'acide 4-benzoylbenzoïque (2mmoles), 412mg de 1,3 dicyclohexylcarbodiimide (DCC) dans 20 ml de $CH_2Cl_2$, et on réalise une agitation pendant 5 minutes.

On ajoute ensuite 264mg (2mmoles) de 4(-2aminoéthyl) phénol ou tyramine, et on agite pendant 5 min.

On ajoute ensuite 80mg de diméthylaminopyridine (DMAP) et on continue l'agitation pendant 5 jours.

On rajoute 50 ml d'eau puis on extrait avec 3 fois 50 ml de $CH_2Cl_2$ . Ensuite $CH_2Cl_2$ est évaporé et le produit obtenu est lavé au méthanol.

**4.3 - Analyse du produit obtenu**

Après évaporation à sec, l'analyse RMN du produit obtenu montre qu'il comprend un mélange de deux produits majoritairement présents, le dérivé phénolique de la benzophénone de la formule ci-dessus, et un sous-produit de formule suivante :

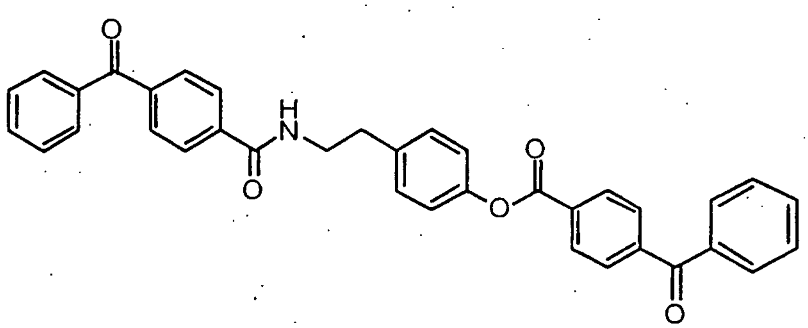

On aboutit à la séparation de ces deux produits par chromatographie sur colonne de silice, éluant dichlorométhane/ hexane/methanol 90/5/5 en volume.
Le spectre RMN du monomère 4-(2-aminoéthyl)phénol-benzophénone est représenté à la figure 21.
On a obtenu 167mg du produit de l'invention recherché, ce qui correspond à un rendement de 22% en mole.

**4.4- Electrochimie et fabrication du polymère poly-[4-(2-aminoéthyl)-phénol]-benzophénone.**

Les propriétés électrochimiques du monomère 4-(2-aminoéthyl)phénol-benzophenone ont été analysées par voltamétrie cyclique sur embout de carbone vitreux.
Le monomère est mis en solution dans du méthanol contenant O,1M de NaOH et 0,1M de $LiClO_4$.
La figure 22 représente en courbe A le signal électrochimique du monomère lors de balayages successifs de potentiel entre 0 et IV relativement à Ag-AgCl.
Lors du premier balayage, un intense pic anodique irréversible est observé à 0,4 V correspondant à l'oxydation des groupements phénoliques.
Au cours des cycles suivants, ce signal diminue puis disparaît.
Ce phénomène est dû à la formation à la surface de l'électrode d'un film polyphénolique isolant, résultant de la polymérisation des radicaux électrongénérés.
Avant cette polymérisation, la figure 22 représente à la courbe B le signal obtenu par cyclage en réduction dans la même solution de monomères. On observe un pic cathodique irréversible correspondant à la réduction des motifs benzophénone.
Après des balayages répétitifs de potentiel entre 0 et 1V, l'électrode est transférée dans une solution électrolytique exempte de monomère et est cyclée dans le domaine de l'électroactivité de la benzophénone, pour obtenir la courbe d'intensité (I) relativement au potentiel V ou E, dite I (V ou E représenté à la figure 23).
Comme on le constate à partir de la figure 23, il apparaît un pic cathodique irréversible correspondant à la réduction des motifs benzophénone qui démontre la présence de ces dernières à la surface de l'électrode et donc la formation d'un film polymère.
Ces résultats démontrent également qu'il est possible d'électrogénérer à la surface d'une électrode, par cyclage électrochimique oxydant, des films de polyphénol substitutés par des motifs bensophénone.
En outre, contrairement aux films polypyrroliques objet des exemples 1 à 3, les films de polyphénols objet du présent exemple 4 sont isolants.

**EXEMPLE 5 - Photogreffage de biomolécules sur les électropolymères photogreffables de l'exemple 4.4.**

**5.1 - Greffage de l'enzyme glucose oxydase**

Les conditions de photogreffage d'une enzyme à la surface du polymère sont identiques à celles utilisées pour le film de polypyrrole benzophénone de l'exemple 3 précédent.
Les électrodes sont immergées et irradiées deux heures dans la solution de glucose oxydase (2mg/ml) désoxygénée.
L'activité de l'enszyme immobilisée à la surface des électrodes a été mesurée par une méthode spectrophotométrique classique des oxydases.
L'oxydation enzymatique du glucose par la glucose oxydase (en abrégé GOD) conduit à la formation de H2O2.

$$\text{glucose + GOD (FAD)} \rightarrow \text{gluconolactone + GOD(FADH}_2\text{) GOD(FADH}_2\text{)} + O_2 \rightarrow \text{GOD (FAD)} + H_2O_2$$

En présence de peroxydase de raifort (HRP), $H_2O_2$ oxyde enzymatiquement l'ortho-toluidine.

Cette espèce chromogène est oxydé en un produit chromophore, espèce absorbante à 425nm.

La variation d'absorbance de l'o-toluidine est suivie en fonction du temps et convertie en activité enzymatique.

Les spectres comportent une partie linéaire pour laquelle l'étape limitante est la production de H2O2 par la glucose oxydate.

Cette dernière étant placée dans des conditions saturantes en glucose, la pente de la partie lénéaire représente la vitesse maximum de la catalyse enzymatique.

Afin de déterminer l'activité enzymatique de l'électrode modifiée, cette dernière est immergée dans une solution de $LiClO_4$ 0,1M, pH 6,8 contenant 250mM de glucose, 2U/ml de péroxydase et 0,2mM d'o-toluidine.

L'absorbance en fonction du.temps, à 425nm, du milieu réactionnel est mesurée en continu et donne la courbe objet de la figure 24 avec en abscisse le temps en minutes et en ordonné l'absorbance exprimée en densité optique (DO).

On observe à partir de la figure 24 que la pente de la partie linéaire est égale à 6m $DO.min^{-1}$, ce qui correspond à une activité enzymatique pour l'électrode modifiée de 68mU.

Cette activité enzymatique démontre la possibilité d'une photoimmobilisaiton de protéine sur un film de polyphénol fonctionnalisé par des motifs benzophénones.

**EXEMPLE 6- Préparation de monomères et polymères de type indole-benzophénone conjugés à des motifs photoactivables et greffage par photoactivation.**

Il est réalisé en pratique la préparation du monomère 3-(2-aminoéthyl)indole-benzophénone.

**6-1 - Formule de ce nouveau composé**

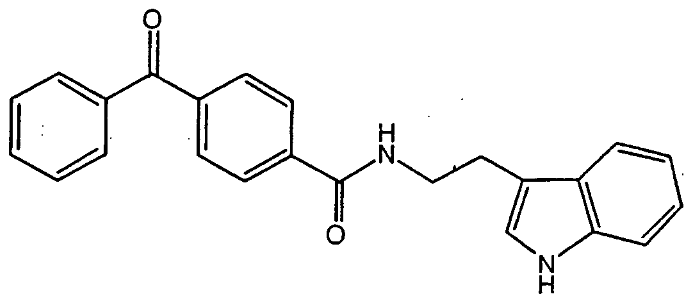

**6-2-Synthése du nouveau composé**

Dans un ballon de 50ml sous argon, on introduit 452mg (2mmoles) d'acide 4-benzoylbenzoïque, 412mg de DCC dans 20ml de $CH_2Cl_2$ et on agite pendant 30 minutes.

On ajoute 320mg de 3-(2-aminoéthyl)indole ou tryptamine et on continue l'agitation pendant 5 minutes.

On ajoute ensuite 80mg de DMAP et on agite pendant 1 semaine.

On rajoute 50 ml d'eau puis on extrait avec 3 fois 50 ml de $CH_2Cl_2$. La phase organique est séchée sur du Na2SO4 puis le solvent est évaporé dans un évaporateur rotatif sous pression réduite et un lavage à l'éthanol puis une chromatographie sur silice (dichlorométhane/ hexane/méthanol : 86/10/4 en volume) sont effectués.

Le rendement du produit de la formule indiquée sous 6.1 est de 68% en mole.

**6-3 - Analyse RMN**

On procède à une analyse RMN classique, et le spectre RMN est donné en figure 25.

**6-4 - Electrochimie de préparation du polymère polyindole-benzophénone.**

De manière similaire aux exemples précédents, on réalise une étude électrochimie par voltamétrie cyclique du monomère (2mM) dans l'acétonitrile, qui indique un prépic à 0,9v suivi d'un pic anodique irréversible à 1,16v correspondant à l'oxydation irréversible du groupement indole.

En réduction, un système de pics réversibles est observé à $E_{1/2}$=-1,8V.

Ce système rédox correspond à la réduction du motif benzophénone.

La modification de la surface de l'électrode est réalisée par électrolyse au potentiel contrôle de 0,8V ou par des balayages répétitifs de potentiel dans le domaine 0-0,9V.

Après transfert dans une solution d'acétonitrile exempte de monomère, le voltammograme de l'électrode modifiée présente un système réversible à $E_{1/2}$=-0,6V dû à l'électroactivité du film de polyindole ainsi qu'un système réversible à $E_{1/2}$=-1,8V correspondant aux motifs benzophénone immobilisés.

Ceci démontre la formation d'un film polymère de polyindole contenant des groupes de benzophénone.

Par conséquent, la fonctionnalisation de la benzophénone par un motif indole électropolymérisable permet d'obtenir des films polymères de polyindole par électro-oxydation.

**EXEMPLE 7 -Préparation de monomères et polymères de type vinyl-benzophénone conjugés à des motifs photoactivables et greffage par photoactivation.**

Comme exemple de monomère de type vinyl-benzophénone, on prépare en pratique le monomère vinylaniline-benzophénone.

**7-1- Formule de ce nouveau composé**

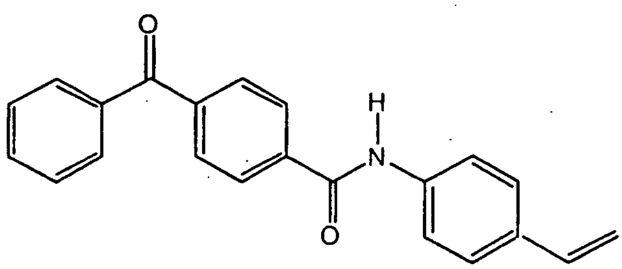

**7-2 - Synthèse de ce nouveau composé**

Dans un ballon de 50ml sous argon, on introduit 452mg (2mmoles) d'acide 4-benzoylbenzoïque, 412mg de DCC dans 20ml de $CH_2Cl_2$ et on agite pendant 30 minutes.

On ajoute ensuite 267mg de vinylaniline (2,2mmoles), on agite encore 5 minutes.

On ajoute ensuite 80mg de DMAP, et on ajoute encore pendant 5 jours.

On rajoute 50 ml d'eau puis on extrait avec 3 fois 50 ml de $CH_2Cl_2$. La phase organique est séchée sur du Na2SO4.

On réalise une chromatographie sur silice (dichlorométhane/hexane/méthanol : 86/10/4 en volume) et on isole le produit vinylaniline-benzophénone de la formule indiquée sous 7.1 avec un rendement de 22%.

**7-3 - Analyse RMN**

On réalise une analyse par RMN de manière classique et on obtient le spectre RMN de la figure 26.

**7-4- Electrochimie de préparation du polymère polyvinyl-benzophénone**

De manière similaire à la préparation des polymères réalisés dans les exemples précédents, le voltamograme du vinyl-benzophénone, 2mmoles dans l'acétonitrile, montre clairement dans le domaine des potentiels négatifs, un pic cathodique irréversible à -1,88V correspondant à la réduction du motif benzophénone.

Cette réduction est suivie d'un transfert électronique conduisant à la formation du radical vinylique siège de la polymérisation.

Des balayages répétitifs de potentiel dans le domaine : 0-2,4V entraînent la formation d'un film polymère de polyvinyle benzophénone à la surface d'une électrode.

Outre l'électroactivité de la benzophénone, l'électrode modifiée exhibe un système de pics quasi-réversible vers -0,15 V dû à l'électroactivité du film polyvinylique fonctionnalisé.

Par conséquent, la fonctionnalisation du motif photoactivable par un groupement vinyl constitue une autre voie

de polymérisation électrochimique des benzophénones sous forme de films polyvinyliques.

## PARTIE III - PREPARATION DE MONOMERES PUIS DE POLYMERES COMPRENANT D'AUTRES MOTIFS PHOTOACTIVABLES.

Comme exemple d'autres motifs photoactivables, on exemplifie ici les groupements azides.
Dans l'exemple préféré, le groupement azide sera fonctionnalisé à un pyrrole comme motif électropolymérisable.

## EXEMPLE 8 - PREPARATION D'UN MONOMERE DE TYPE AZIDOPYRROLE, PAR EXEMPLE 4-FLUORO-3-NITRO-PHENYLAZIDE-PYRROLE

### 8.1 - Formule du nouveau composé

La formule de ce nouveau composé est donné ci-après :

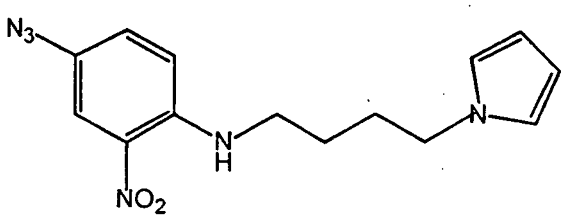

### 8.2 Synthèse de ce nouveau composé dans l'obscurité

Dans un ballon de 25 ml, on introduit 207 mg de aminobutylpyrrole (1,5 mmoles) dans 5 ml de THF sec. On ajoute ensuite 250 mg (1,37 mmoles) de 4-fluoro-3-nitro-phénylazide dans 5 ml de THF sec, sous agitation pendant 30 minutes. On réalise l'évaporation du THF, la dissolution du précipité obtenu dans 7 ml de $H_2O$. On ajoute ensuite 7 ml de NaOH 1M.
On réalise ensuite une extraction par deux fois 15 ml d'acétate d'éthyle puis on réalise un séchage de la solution d'acétate d'éthyle sur $Na_2SO_4$ puis une chromatographie de celle-ci dans les mêmes conditions de chromatographie que dans les exemples précédents, pour obtenir le produit de la formule indiquée sous 8.1 avec un rendement de 54 % en mole.

### 8.3 - Analyse RMN

Après chromatographie, la pureté du produit et sa formule chimique a été confirmée par RMN. Le spectre RMN est donné en figure 27.

### 8.4 - Electrochimie de synthèse du polymère polypyrrole comportant un groupe azide photoactivable

Les propriétés électrochimiques du monomère ont été analysées par voltamétrie cyclique sur embout de carbone vitreux. Le monomère est mis en solution à l'abri de la lumière dans l'acétonitrile (1,5 mM). Comme le montre la figure 28, le voltammogramme présente dans la partie anodique un pic irréversible à +0,95 V/Ag/$Ag^+$ correspondant à l'oxydation du groupe pyrrole.
Dans le domaine cathodique, plusieurs vagues de réduction irréversible (-1,4 ; -1,5 et -1,7 V/Ag/$Ag^+$) sont observées, pouvant correspondre à des réductions successives du groupement nitro.
La formation de films pyrroliques a été réalisée par balayages répétitifs de potentiel entre 0 et 0,90 V/Ag/$Ag^+$. Cette formation est illustrée par l'apparition et la croissance d'un système rédox à +0,4 V/Ag/$Ag^+$ caractéristique de l'électroactivité des chaînes polypyrroliques et la courbe obtenue fait l'objet de la figure 29, courbe A.
Les films peuvent également être formés par électrolyse à potentiel contrôle (0,9 V/Ag/$Ag^+$). La courbe obtenue est représentée à la figure 30, courbe B, qui présente le signal de l'électrode après une électro-oxydation de 2 mC et son transfert dans une solution électrolytique exempte de monomère. Le voltamogramme de cette électrode montre clairement l'électroactivité classique des films pyrroliques dans le domaine anodique.
Ces premiers résultats montrent qu'il est possible d'électrogénérer à la surface de l'électrode des films de

polymère à partir d'une solution organique de monomère azidopyrrole. Les films obtenus de façon reproductible sont hydrophobes, résistants et d'épaisseur contrôlable.

**8.5 - Greffage d'une entité biologique, par exemple l'enzyme glucose oxydase (GOD)**

Comme pour les motifs benzophénone, les azides, de préférence les arylazides, sont précurseurs de la photogénération de radicaux.

Par photolyse, la formation d'intermédiaires nitrènes hautement réactifs conduit à la formation de liaison covalente entre intermédiaire photogénéré et biomolécule.

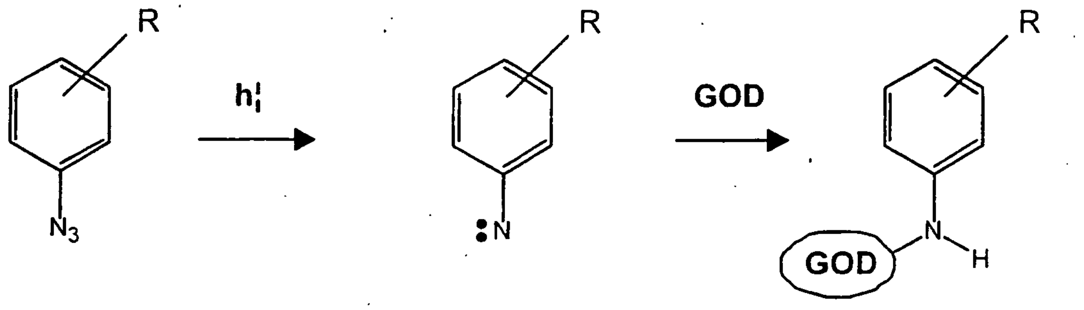


Des films de polypyrrole ont été formés par électrolyse (2 mC) à potentiel imposé, sur électrode de platine. Les polymères obtenus, protégés de la lumière, ont été transférés dans une solution aqueuse d'enzyme glucose oxydase ou GOD à raison de 1 mg/ml et irradiés pendant deux heures sous une lumière (300-350 nm) issue d'une lampe à mercure de 250 W

**8.5-A Mesure de l'activité enzymatique des électrodes irradiées**

Après rinçage des électrodes, leur activité enzymatique a été déterminée selon la méthode utilisée pour les électrodes modifiées par du polypyrrole benzophénone.

La pente de la partie linéaire de la courbe d'absorbance en fonction du temps de l'o-toluidine est égale à 0,38 mDo/min, ce qui correspond à une activité enzymatique de l'électrode de 22 mU/cm$^2$. Cette activité enzymatique du film polypyrrolique fonctionnalisé par des motifs azido démontre la présence de molécules d'enzyme immobilisé par photogreffage.

**8.5-B Dosage du glucose**

Le dosage ampérométrique du glucose a été entrepris en milieu aqueux avec les électrodes greffées à + 600 Mv/Ag/AgCl afin d'oxyder à la surface de l'électrode les molécules de $H_2O_2$ enzymatiquement générées. Une courbe d'étalonnage du glucose est présentée en figure 31.

Comme on l'observe à partir de la figure 31, la réponse ampérométrique de l'électrode modifiée à des ajouts de glucose présente une partie linéaire de pente égale à 40 μA/M/cm$^2$ pour une concentration inférieure à 8 Mm. Au-delà de cette concentration, la courbe s'incurve pour tendre vers une valeur correspondant au courant maximum du biocapteur ($I_{max}$ = 120 nA) lorsque les enzymes sont saturées en substrat.

**8.4-C Représentation de Lineweaver-Burk**

La forme de la courbe de calibration obtenue est similaire à celle d'une courbe décrivant une catalyse enzymatique de type Michaelis-Menten. En considérant que dans le processus enzymatique de détection du substrat l'étape enzymatique est l'étape limitante, l'intensité de la réponse ampérométrique peut être assimilée à la vitesse de la réaction enzymatique. Dans cette hypothèse, la modélisation de la réponse du biocapteur est réalisée à l'aide de la représentation de Lineweaver-Burk.

$$\frac{1}{I} = \frac{1}{I_{max}} + \frac{K_m^{app}}{I_{max}} \frac{1}{C}$$

dans laquelle :

I : intensité de la réponse ampérométrique du biocapteur
$I_{max}$ : intensité maximale de la réponse ampérométrique du biocapteur en présence d'une concentration saturante de substrat $K_m^{app}$ : constante apparente de Michaëlis, caractéristique de l'ensemble du biomatériau
C : concentration en glucose.

Comme on l'observe à partir de la figure 32, cette modélisation permet d'accéder à la constante apparente de Michaelis-Menten, à savoir $K_m^{app}$.

La valeur $K_m^{app}$ obtenue est égale à 20 mM comme cela résulte de la figure 32 et est similaire à celle de la constante cinétique de l'enzyme libre en solution. Cette similitude de valeur est représentative de la faible contrainte imposée aux enzymes photogreffées à la surface d'un polymère par rapport à des enzymes encapsulées au sein d'un matériau.

L'ensemble de ces résultats montrent l'efficacité du photogreffage de biomolécules par irradiation des sites azides électropolymérisés.

Il apparaît donc que le site pyrrolique permet l'obtention, sous électrolyse à potentiel contrôle, d'un film de polymère hydrophobe, résistant et d'épaisseur contrôlable, fonctionnalisés par des motifs azides conservant leur propriété de photogreffage sous irradiation.

**Revendications**

1. Monomère comprenant au moins une entité électropolymérisable et au moins un motif à la fois photoactivable et photogreffable, choisi parmi :

■ le monomère pyrrolbenzophénone de formule :

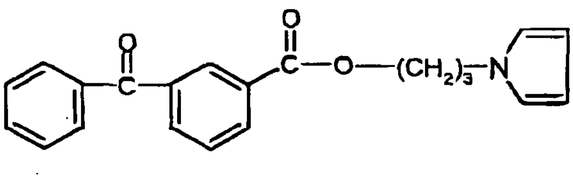

(benzophénone) (pyrrole)

en particulier obtenu par réaction de l'acide 3-benzoylbenzoïque avec le 3-pyrrol-1-propanol,

■ le monomère 4-(2-aminoéthyl)phénol-benzophénone de formule :

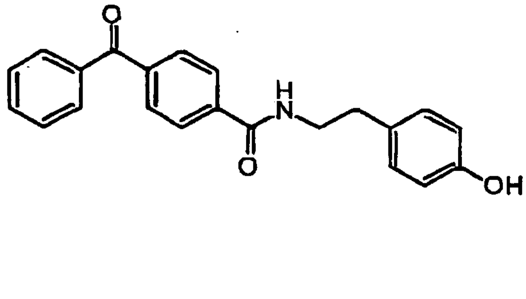

en particulier obtenu par réaction du 4-(2-aminoéthyl)phénol avec l'acide 4-benzoylbenzoïque,
■ le monomère indol-benzophénone de formule :

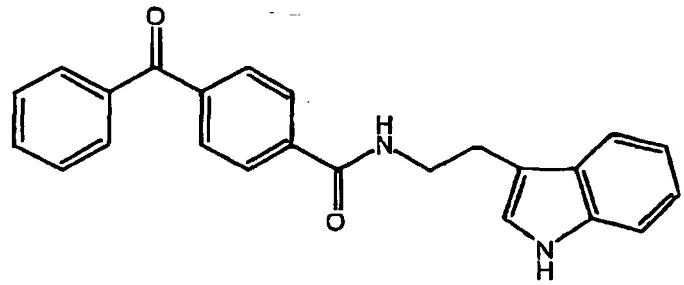

en particulier obtenu par réaction du 3-(2-aminoéthyl)indole avec l'acide 4-benzoylbenzoïque,
■ le monomère vinylaniline-benzophénone de formule :

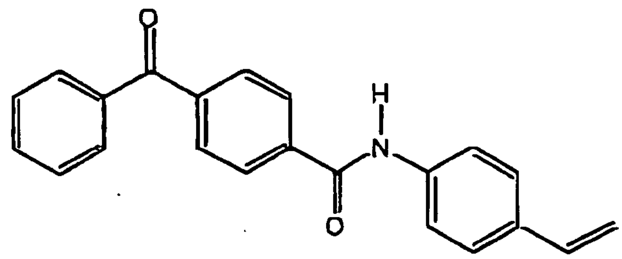

en particulier obtenu par réaction de la vinylaniline avec l'acide 4-benzoylbenaoique,
■ le monomère azidophénylpyrrole de formule :

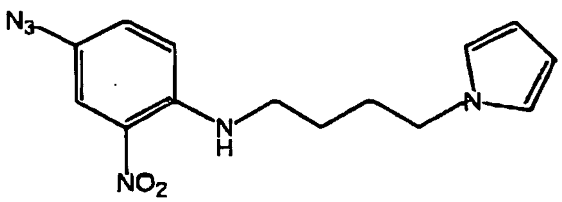

en particulier obtenu par réaction de 4-fluoro-3-nitro-phénylazide avec l'aminobutylpyrrole.

2. Electropolymère photogreffable, **caractérisé en ce qu'**il est préparé à partir d'un monomère tel que définis dans la revendication 1.

3. Electropolymère selon la revendication 2, ou monomère selon la revendication 1, **caractérisé en ce qu'**il est photogreffé avec une substance photogreffable choisie parmi les (macro)molécules biologiques et/ou les chimères de celles-ci et/ou parmi les unités de ces (macro)molécules et/ou de ces chimères.

4. Electropolymère selon la revendication 2, ou monomère selon la revendication 1, **caractérisé en ce qu'**il est photogreffé avec une substance photogreffable choisie parmi les (macro)molécules suivantes : des acides aminés, des oligopeptides, des polypeptides, des protéines, des gluco-protéines, des lipoprotéines, des nucléotides, des oligonucléotides, des polynucléotides type ARN ou ADN, d'origine biologique ou synthétique, des enzymes, des co-enzymes et des vitamines; ou les chimères de biomolécules suivantes: des polyacides nucléiques peptidiques, des sites prosthétiques d'enzymes tels que des métalloporphyrines, des isobacténoclorines, des corrines, des chlorins, des anticorps artificiels à base par exemple de polymères à empreinte.

5. Poly(pyrrolbenzophénone) obtenu à partir du monomère pyrrolbenzophénone de formule :

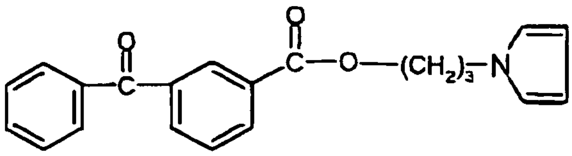

(benzoph´nonc) (pyrrole)

photogreffé avec une substance photogreffable choisie parmi une protéine, de préférence l'albumine de sérum bovin ou humain, une enzyme, de préférence la glucose oxydase ; une biotine, de préférence le système affin avidine-biotine, la thionine ; un anticorps, de préférence un anticorps de lapin (IgG).

6. Polymère de polyphénolbenzophénone obtenu à partir du monomère 4-(2-aminoéthyl)phénol-benzophénone de formule:

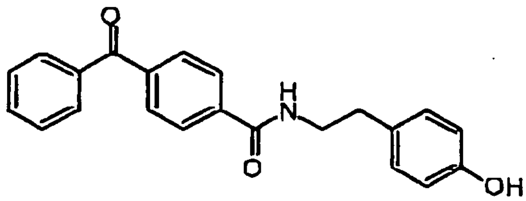

ou du monomère azidophénylpyrrole de formule :

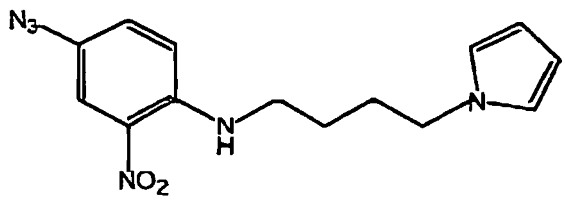

photogreffé avec une enzyme photogreffable de préférence la glucose oxydase.

7. Procédé de fabrication de biocapteurs électroniques du type de ceux comprenant au moins un (micro)transducteur comportant au moins une membrane sensible porteuse de sondes de reconnaissance spécifique de molécules cibles à analyser, **caractérisé en ce que** l'on réalise la membrane sensible fixée sur le support transducteur à partir d'au moins un polymère selon la revendication 2 et **en ce que** l'on greffe (de préférence de manière localisée), les sondes de reconnaissance spécifique sur les motifs photoactivables du polymère par photoactivation.

8. Biocapteur électronique du type de ceux comprenant au moins un (micro)transducteur comportant au moins une membrane sensible porteuse de sondes de reconnaissance spécifique **caractérisé en ce que** la membrane sensible comprend au moins un polymère selon la revendication 2, sur lequel sont photogreffés (de préférence de manière localisée), des substituants qui correspondent aux sondes de reconnaissance spécifique.

9. Support formant électrode, **caractérisé en ce qu'**il comprend à sa surface au moins une couche d'un polymère selon l'une des revendications 2 à 6.

## Claims

**1.** Monomer comprising at least one electropolymerisable entity and at least one group capable of being both light-activated and grafted on using light, chosen from among:

• the pyrrolbenzophenone monomer of formula:

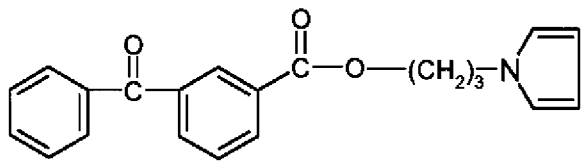

(benzophenone) (pyrrol )

in particular obtained by reacting 3-benzoylbenzoic acid with 3-pyrrol-1-propanol,

• the 4-(2-aminoethyl)phenol-benzophenone monomer of formula:

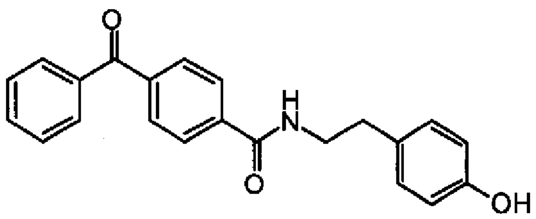

in particular obtained by reacting 4-(2-aminoethyl)phenol with 4-benzoylbenzoic acid,

• the indol-benzophenone monomer of formula:

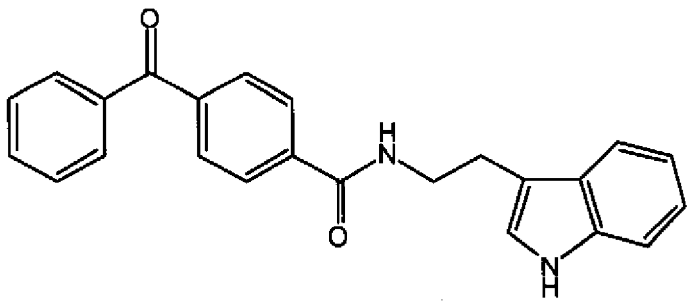

in particular obtained by reacting 3-(2-aminoethyl)indole with 4-benzoylbenzoic acid,

■ the vinylaniline-benzophenone monomer of formula:

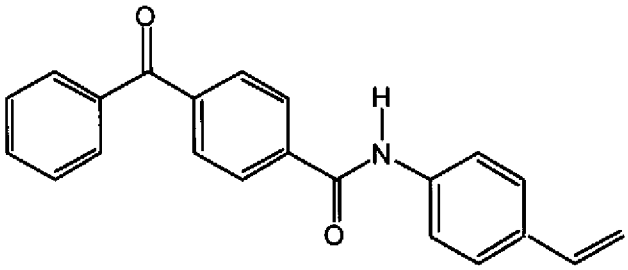

in particular obtained by reacting vinylaniline with 4-benzoylbenzoic acid,

■ the azidophenylpyrrole monomer of formula:

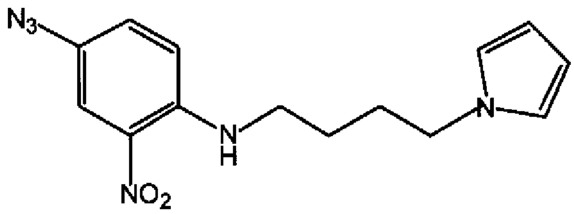

in particular obtained by reacting 4-fluoro-3-nitro-phenylazide with aminobutylpyrrole.

**2.** Electropolymer capable of being grafted on by light, **characterised in that** it is prepared from a monomer such as defined in claim 1.

**3.** Electropolymer according to claim 2, or monomer according to claim 1, **characterised in that** it is grafted on using light with substance capable of being grafted on by light, that is chosen from among biological (macro)molecules and/or chimera of these and/or units of these (macro)molecules and/or these chimera.

**4.** Electropolymer according to claim 2, or monomer according to claim 1, **characterised in that** it is grafted on using light with substance capable of being grafted on by light, that is chosen from among the following (macro)molecules: amino acids, oligopeptides, polypeptides, proteins, glucoproteins, lipoproteins, nucleotides, oligonucleotides, RNA or DNA type polynucleotides of biological or synthetic origin, enzymes, coenzymes and vitamins; or chimera of the following biomolecules: peptidic nucleic polyacids, enzyme prosthetic sites such as metalloporphyrines, isobacteriochlorines, corrines, chlorines, artificial antibodies based, for example, on the polymer to be imprinted.

**5.** Poly(pyrrolbenzophenone) obtained from the pyrrole-benzophenone monomer of formula:

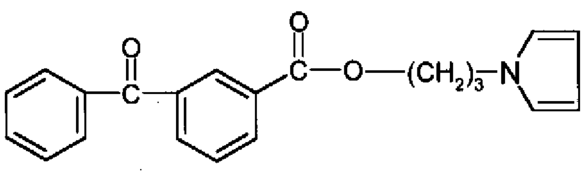

(benzophenone) (pyrrol )

grafted on using light with a light-induced grafting substance chosen from a protein, preferably bovine or human serum albumin, an enzyme, preferably glucose oxidase, a biotine, preferably the avidine-biotine affine system, thionine, an antibody, preferably rabbit antibody (IgG).

**6.** Polyphenol-benzophenone polymer obtained from the 4-(2-aminoethyl)phenol-benzophenone monomer of formula:

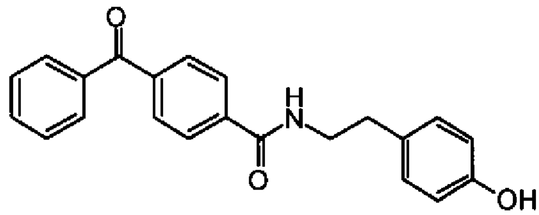

or the azidophenylpyrrole monomer of formula:

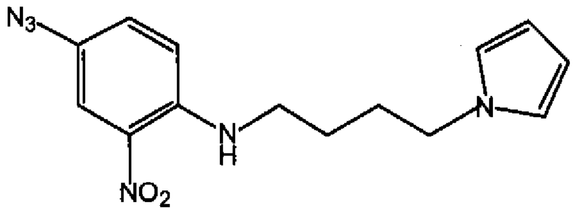

grafted on using light with enzyme capable of being grafted on by light, preferably glucose oxidase.

7. Process for the manufacture of electronic biosensors of the type incorporating at least one (micro)transducer including at least one sensitive membrane carrying identification probes specific to the target molecules to be analysed, **characterised in that** the sensitive membrane is fixed on the transducer support by means of at least one polymer according to claim 2, and **in that** the specific identification probes are grafted (preferably in a localised manner) onto groups of the polymer capable of being light-actived by means of light-induced activation.

8. Electronic biosensor of the type incorporating at least one (micro)transducer including at least one sensitive membrane carrying specific identification probes, **characterised in that** the sensitive membrane includes at least one polymer according to claim 2, onto which substituents corresponding to specific identification probes are grafted on using light (preferably in a localised manner).

9. Electrode-forming support, **characterised in that** its surface includes at least one polymer layer according to one of claims 2 to 6.

**Patentansprüche**

1. Monomer, umfassend wenigstens eine elektropolymerisierbare Einheit und wenigstens ein Motiv, das gleichzeitig photoaktivierbar und photopfropfbar ist, gewählt unter:

   - dem Pyrrolbenzophenonmonomer mit der Formel:

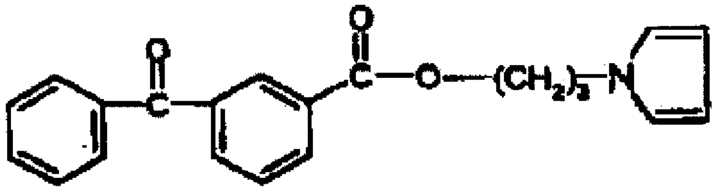

(Benzophenon) (Pyrrol)

insbesondere erhalten durch Reaktion der 3-Benzoylbenzoesäure mit 3-Pyrrol-1-Propanol,
- dem Monomer 4-(2-Aminoethyl)-Phenol-Benzophenon mit einer Formel:

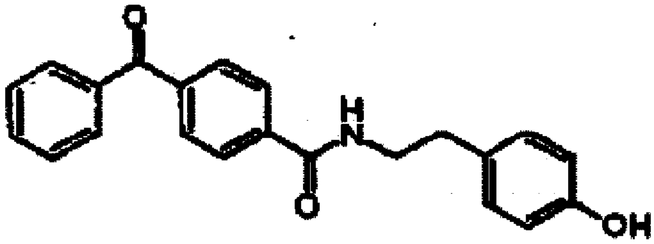

insbesondere erhalten durch Reaktion des 4-(2-Aminoethyl)-Phenols mit 4-Benzoylbenzoesäure,
- dem Monomer Indol-Benzopehnon mit einer Formel:

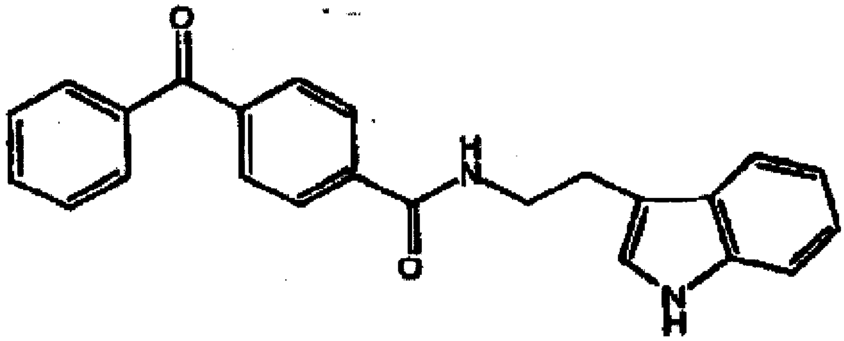

insbesondere erhalten durch Reaktion des 3-(2-Aminoethyl)-Indols mit 4-Benzoylbenzoesäure,
- dem Monomer Vinylanilin-Benzophenon mit einer Formel:

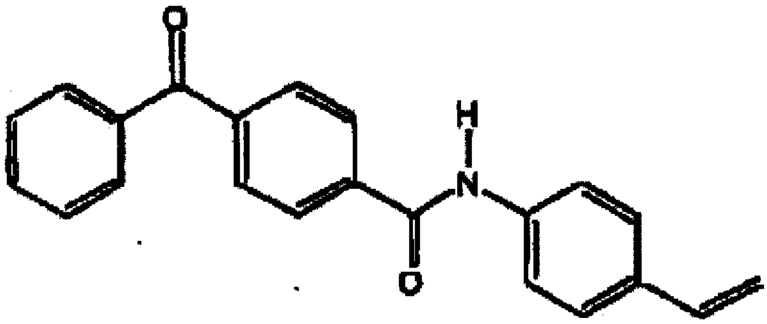

insbesondere erhalten durch Reaktion des Vinylanilins mit 4-Benzoylbenzoesäure,
- dem Monomer Azidophenylpyrrol mit einer Formel:

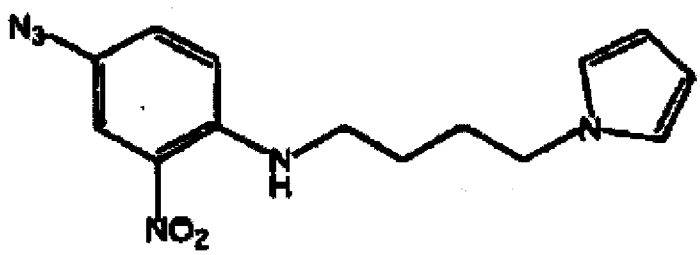

insbesondere erhalten durch Reaktion des 4-Fluor-3-Nitro-Phenylazids mit Aminobutylpyrrol.

2. Photopfropfbares Elektropolymer, **dadurch gekennzeichnet, daß** es hergestellt wird aus einem Monomer wie definiert in Anspruch 1.

3. Elektropolymer nach Anspruch 2 oder Monomer nach Anspruch 1, **dadurch gekennzeichnet, daß** es photogepfropft wird mit einer photopfropfbaren Substanz, gewählt unter den biologischen (Makro-)Molekülen und/oder den Chimären von jenen und/oder unter den Einheiten dieser (Makro-)Moleküle und/oder dieser Chimären.

4. Elektropolymer nach Anspruch 2 oder Monomer nach Anspruch 1, **dadurch gekennzeichnet, daß** es photogepfropft ist mit einer photopfropfbaren Substanz, gewählt unter den folgenden (Makro-)Molekülen: Aminosäuren, Oligopeptide, Polypeptide, Proteine, Glucoproteine, Lipoproteine, Nukleotide, Oligonukleotide, Polynukleotide vom Typ RNA oder DNA, biologischen oder synthetischen Ursprungs, Enzyme, Coenzyme und Vitamine; oder die Chimären folgender Biomoleküle: peptidische Polynukleinsäuren, prostethische Stellen von Enzymen wie Metalloporphyrine, Isobacteriochlorine, Corrine, Chlorine, künstliche Antikörper zum Beispiel auf Basis von Polymeren mit Abdruck.

5. Poly-(Pyrrolbenzophenon) erhalten aus dem Pyrrolbenzophenonmonomer mit einer Formel:

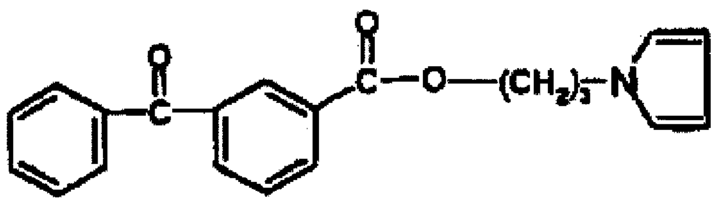

(Benzophenon) (Pyrrol)

photogepfropft mit einer photopfropfbaren Substanz, gewählt unter einem Protein, vorzugsweise Rinder- oder Humanserumalbumin, einem Enzym, vorzugsweise Glucoseoxydase, einem Biotin, vorzugsweise dem affinen Avidin-Biotin-System, Thionin, einem Antikörper, vorzugsweise einem Kaninchenantikörper (IgG).

6. Polyphenolbenzophenonpolymer, erhalten aus dem Monomer 4-(2-Aminoethyl)-Phenol-Benzophenon mit einer Formel:

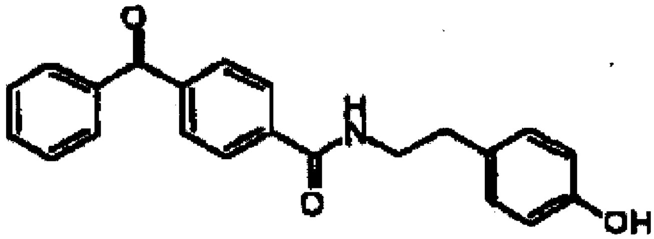

oder dem Monomer Azidophenylpyrrol mit einer Formel:

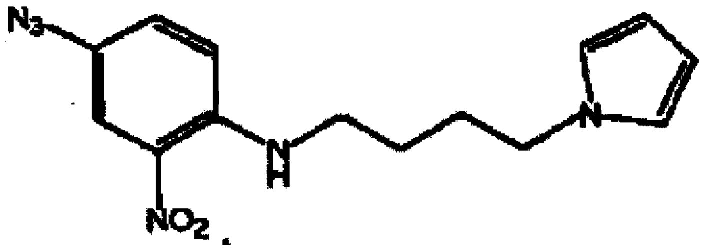

photogepfropft mit einem photopfropfbaren Enzym, vorzugsweise der Glucoseoxidase.

**7.** Verfahren zur Herstellung von elektronischen Biosensoren des Typs von jenen, die wenigstens einen (Mikro-) Meßwertgeber umfassen, umfassend wenigstens eine sensible Membran, die Träger von Sonden zur spezifischen Erkennung von Zielmolekülen ist, die zu analysieren sind, **dadurch gekennzeichnet, daß** man die sensible Membran fixiert auf dem Meßwertgeberträger ausführt, ausgehend von wenigstens einem Polymer nach Anspruch 2, und daß man (vorzugsweise lokalisiert) die Sonden zur spezifischen Erkennung auf die photoaktivierbaren Motiven des Polymers durch Photoaktivierung aufpfropft.

**8.** Elektronischer Biosensor des Typs von jenen, die wenigstens einen (Mikro-)Meßwertgeber umfassen, umfassend wenigstens eine empfindliche Membran, die Träger von Sonden zur spezifischen Erkennung ist, **dadurch gekennzeichnet, daß** die empfindliche Membran wenigstens ein Polymer nach Anspruch 2 umfaßt, auf welche (vorzugsweise lokalisiert) Substituenten photogepfropft sind, die den Sonden zur spezifischen Erkennung entsprechen.

**9.** Träger, der eine Elektrode bildet, **dadurch gekennzeichnet, daß** er an seiner Oberfläche wenigstens eine Schicht eines Polymers nach einem der Ansprüche 2 bis 6 umfaßt.

FIG.1

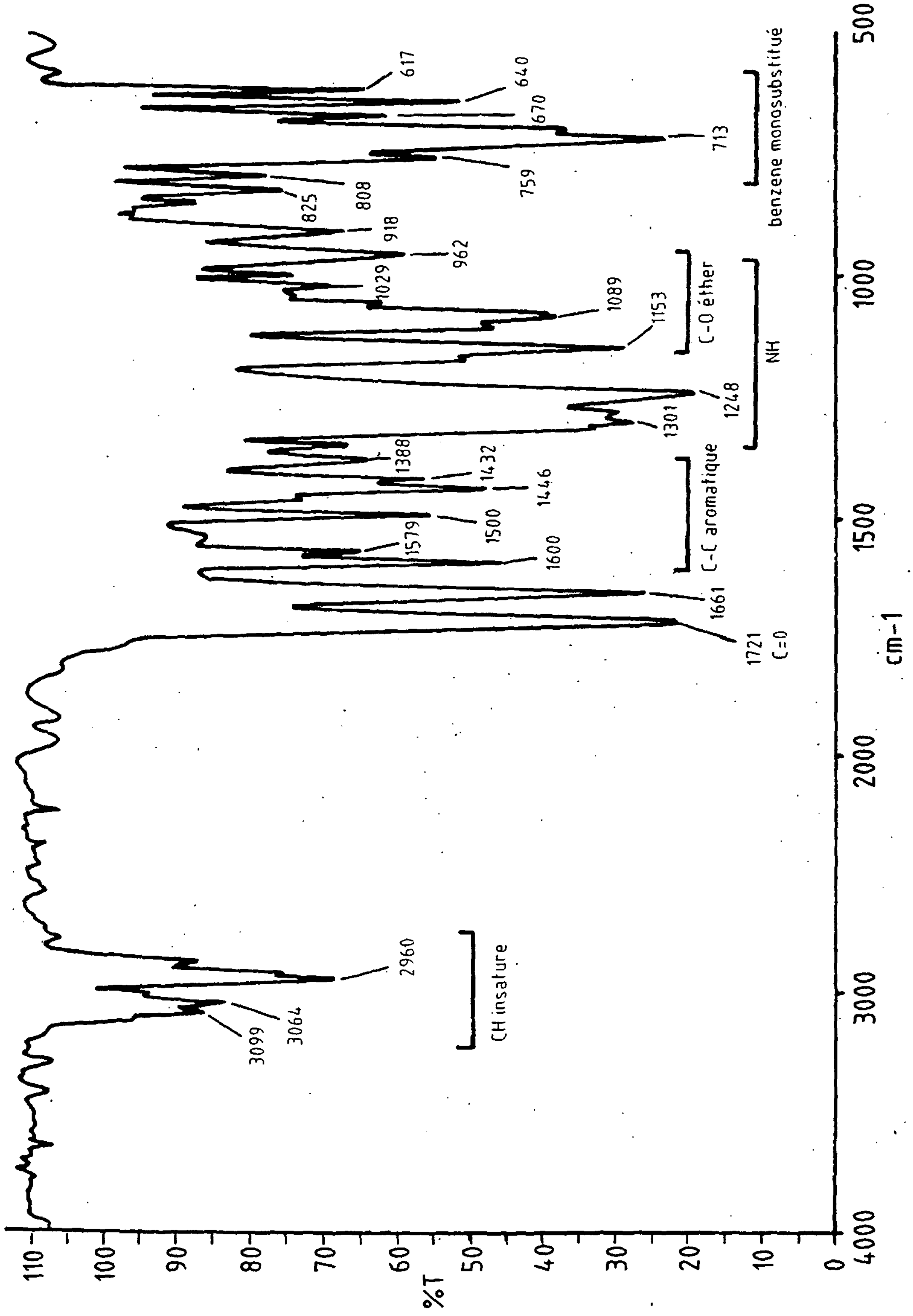
%T
110
100
90
80
70
60
50
40
30
20
10
0
4000
3000
2000
1500
1000
500
cm-1
3099
3064
2960
CH insature
1721
C=O
1661
1600
1579
1500
1446
1432
1388
C-C aromatique
1301
1248
NH
1153
1089
C-O éther
1029
962
918
825
808
759
713
670
640
617
benzene monosubstitué

FIG.2

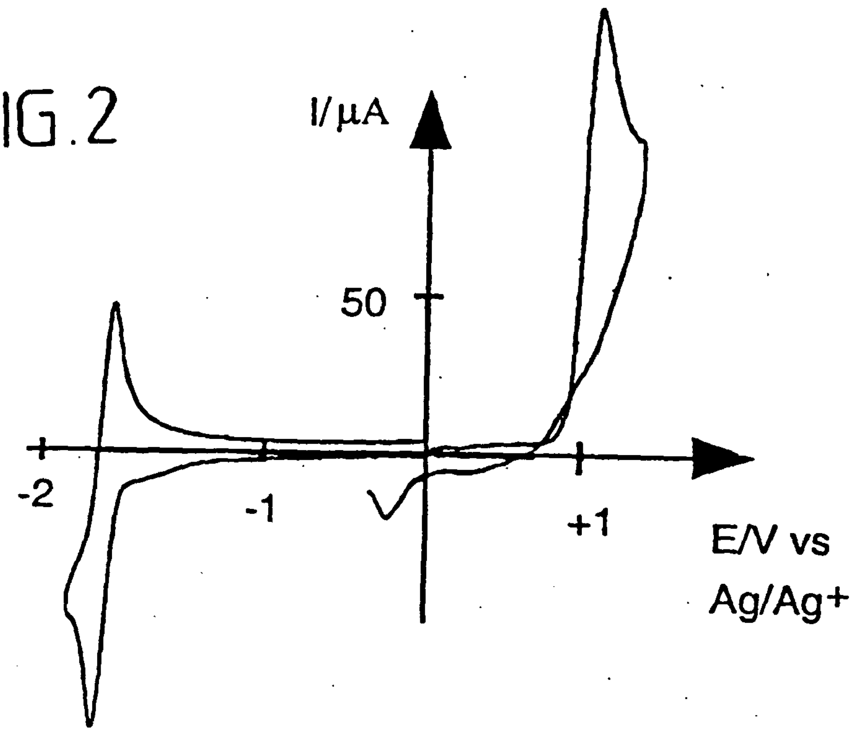


FIG.3

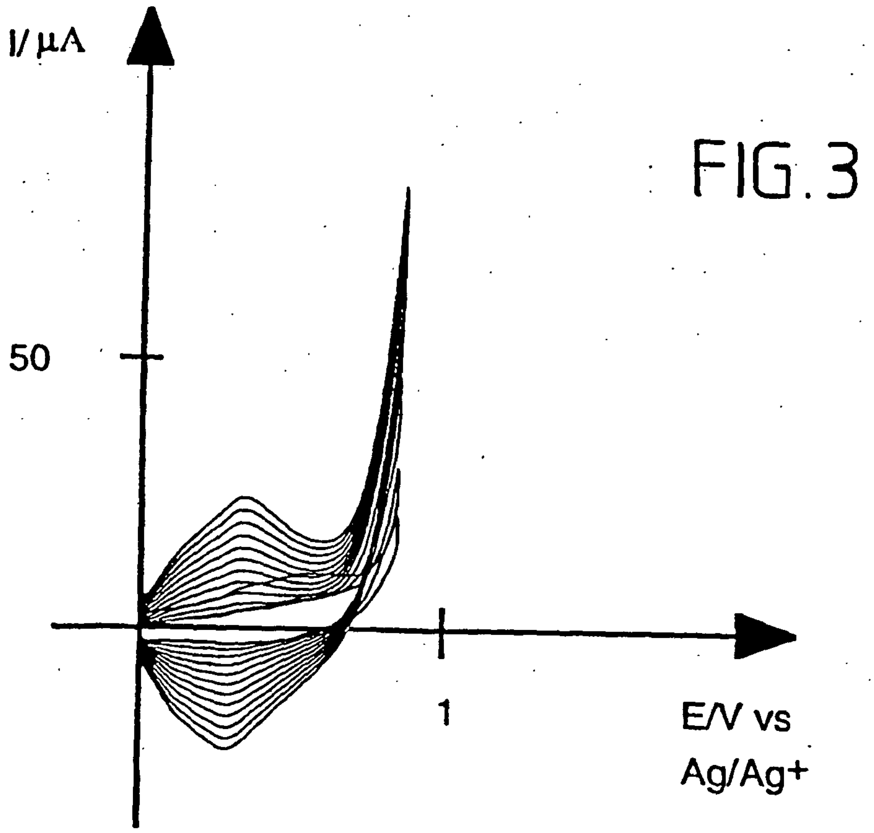

FIG.4

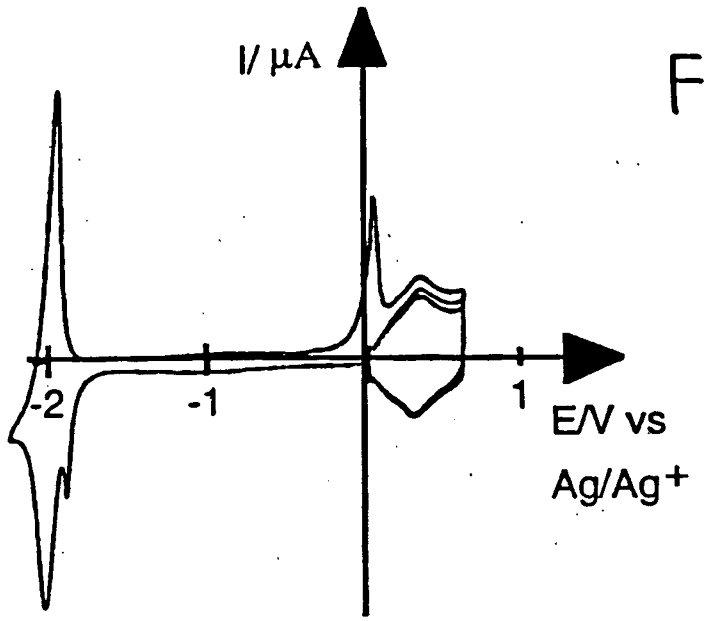
I/ µA
-2
-1
1
E/V vs
Ag/Ag+

FIG.5

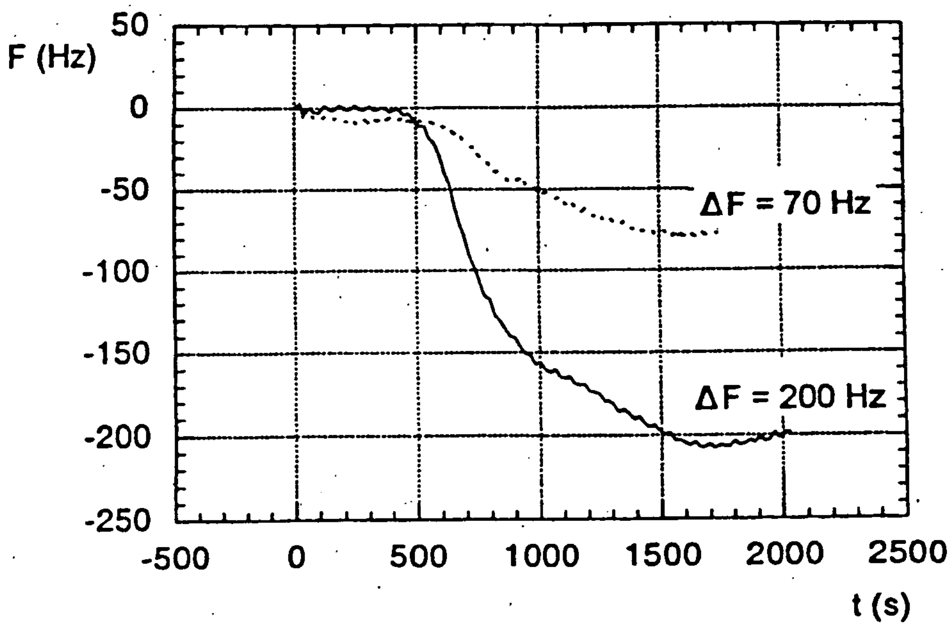
F (Hz)
50
0
-50
-100
-150
-200
-250
-500
0
500
1000
1500
2000
2500
t (s)
ΔF = 70 Hz
ΔF = 200 Hz

FIG.6

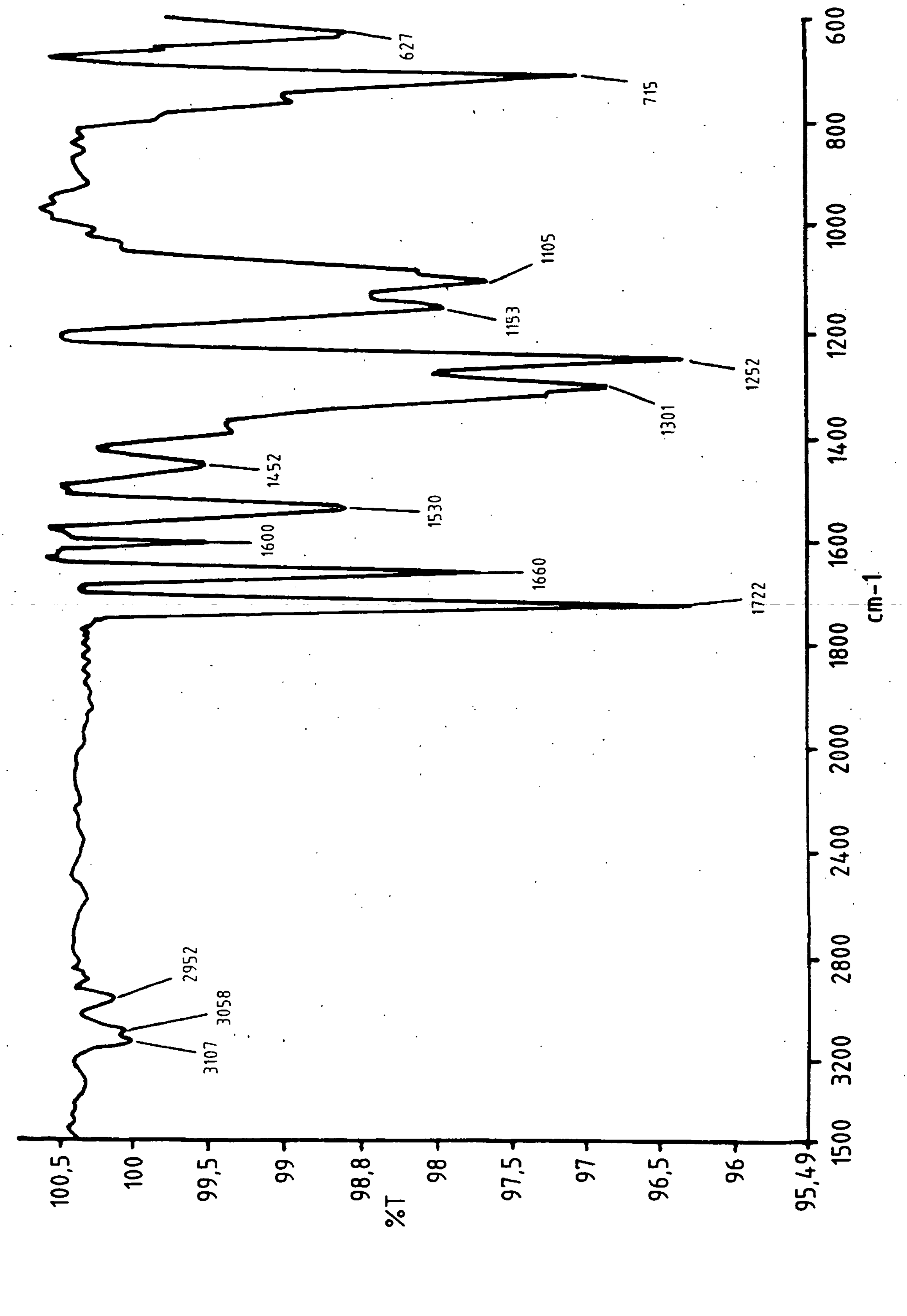
627
715
1105
1153
1252
1301
1452
1530
1600
1660
1722
2952
3058
3107
600
800
1000
1200
1400
1600
1800
2000
2400
2800
3200
3500
cm-1
100,5
100
99,5
99
98,8
98
97,5
97
96,5
96
95,49
%T

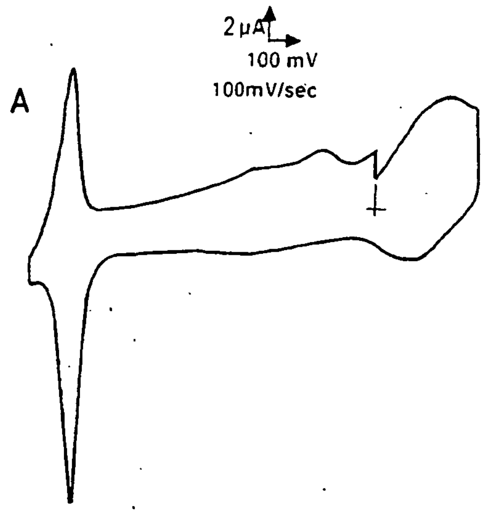


FIG.7

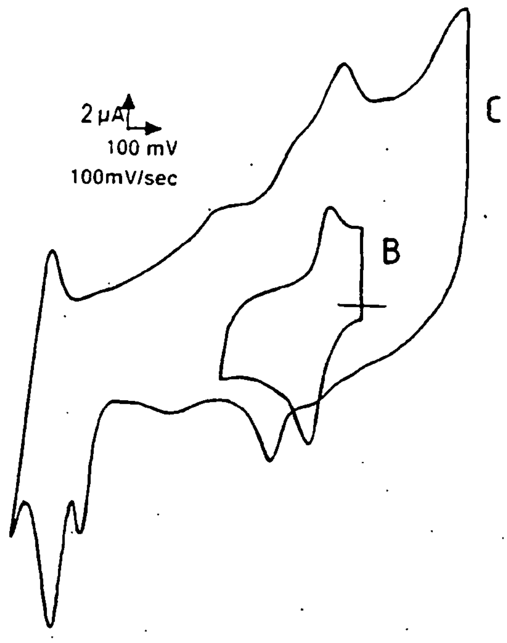


FIG.8

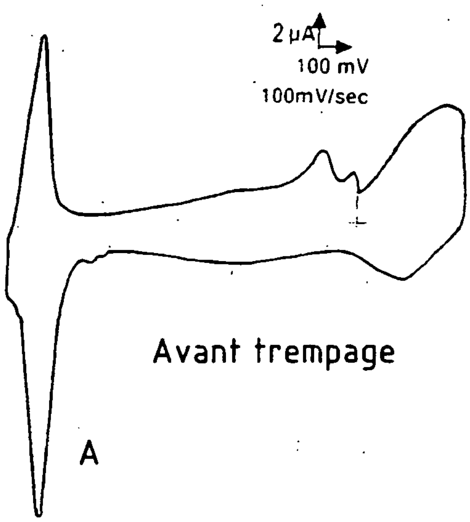


FIG.9

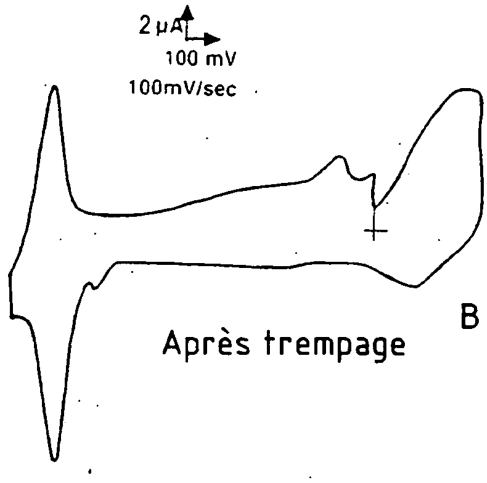


FIG.10

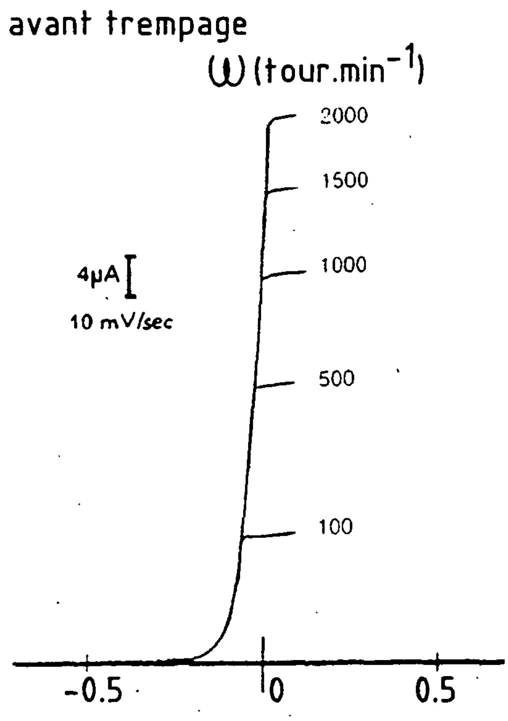


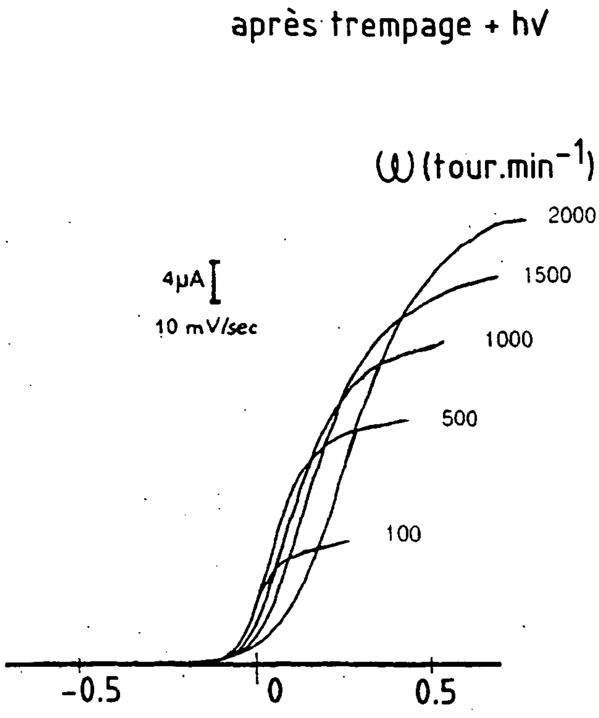


Potentiel (V/Ag/Ag$^{+}$)

FIG.11

FIG.12

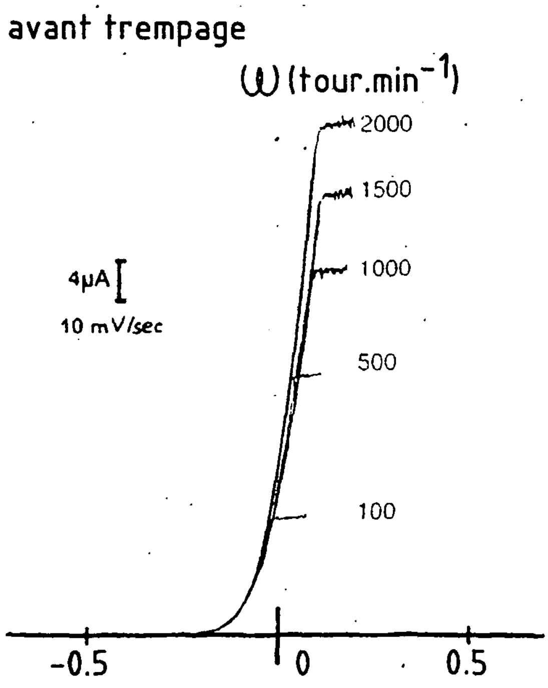


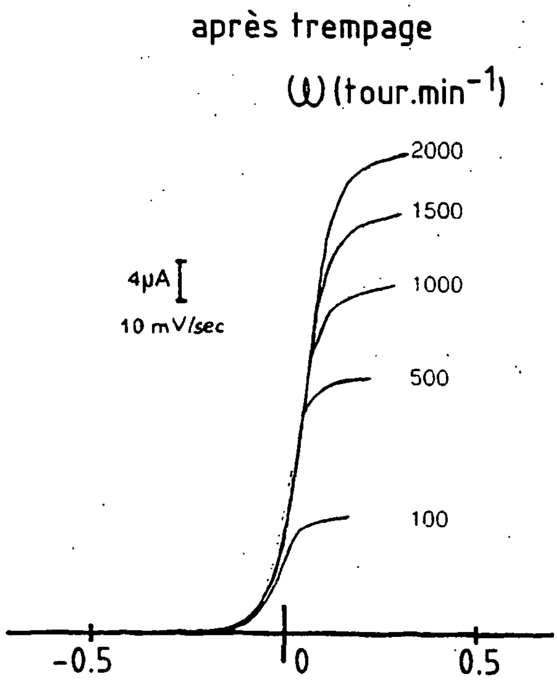


FIG.13

Potentiel (V/Ag/Ag$^{+}$)

FIG.14

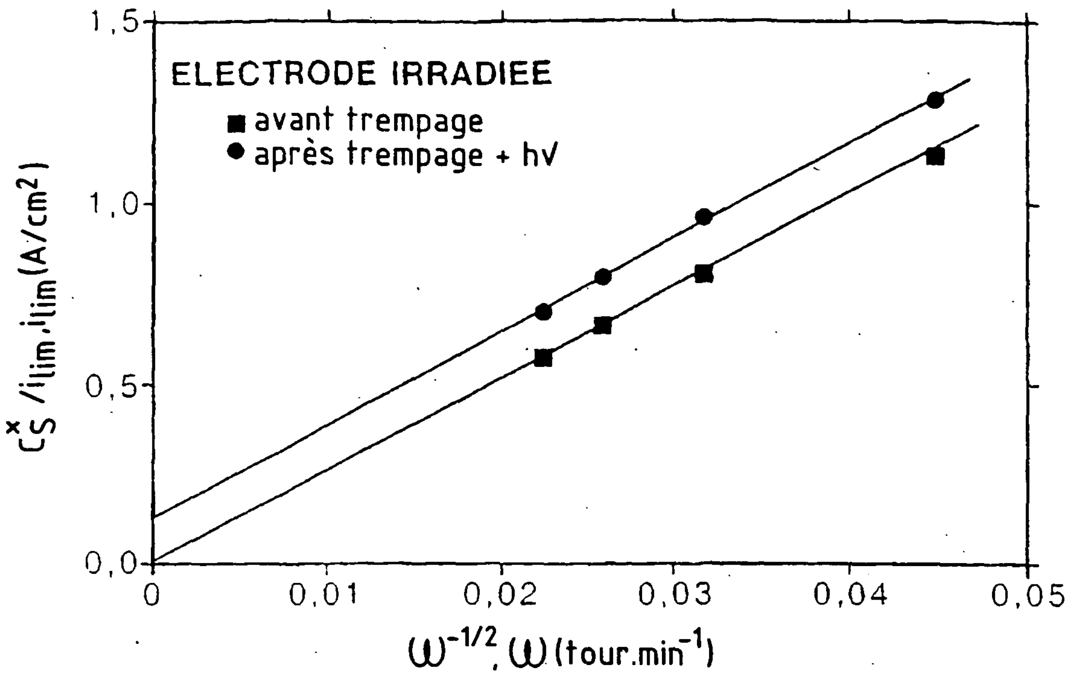
ELECTRODE IRRADIEE
avant trempage
après trempage + hV
1,5
1,0
0,5
0,0
0
0,01
0,02
0,03
0,04
0,05
$C_S^x/i_{lim}, i_{lim}$ (A/cm²)
$\omega^{-1/2}$, $\omega$ (tour.min⁻¹)

FIG.15

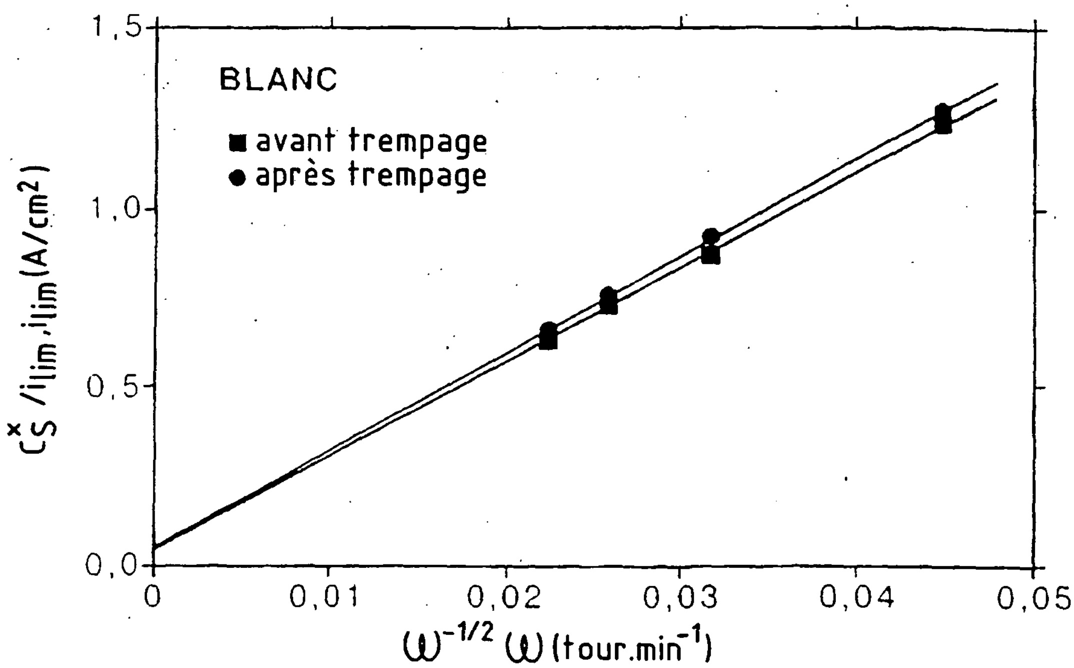
BLANC
avant trempage
après trempage
1,5
1,0
0,5
0,0
0
0,01
0,02
0,03
0,04
0,05
$C_S^x/i_{lim}, i_{lim}$ (A/cm²)
$\omega^{-1/2}$ $\omega$ (tour.min⁻¹)

FIG.16

Avant trempage

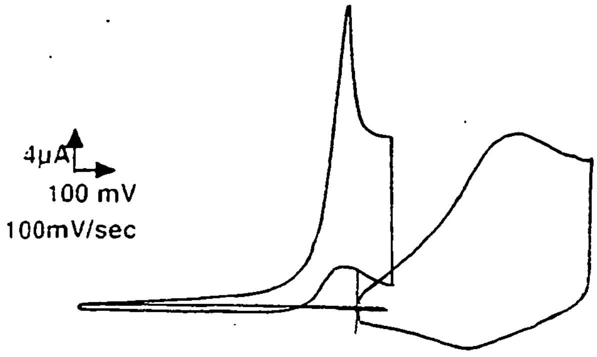


FIG.17

Après trempage + hV

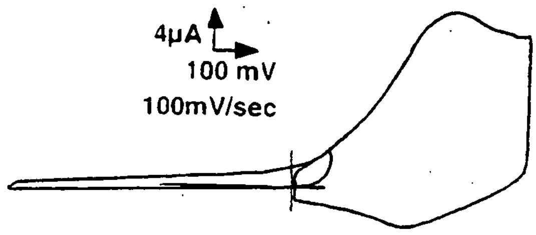


FIG.18

Avant trempage

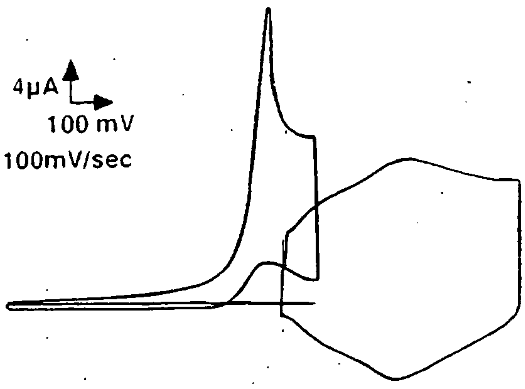


FIG.19

Après trempage
sans irradiation

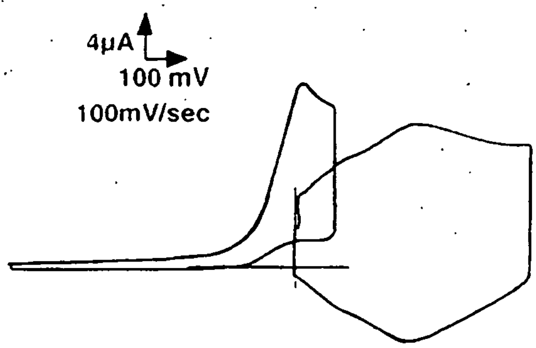


FIG.20

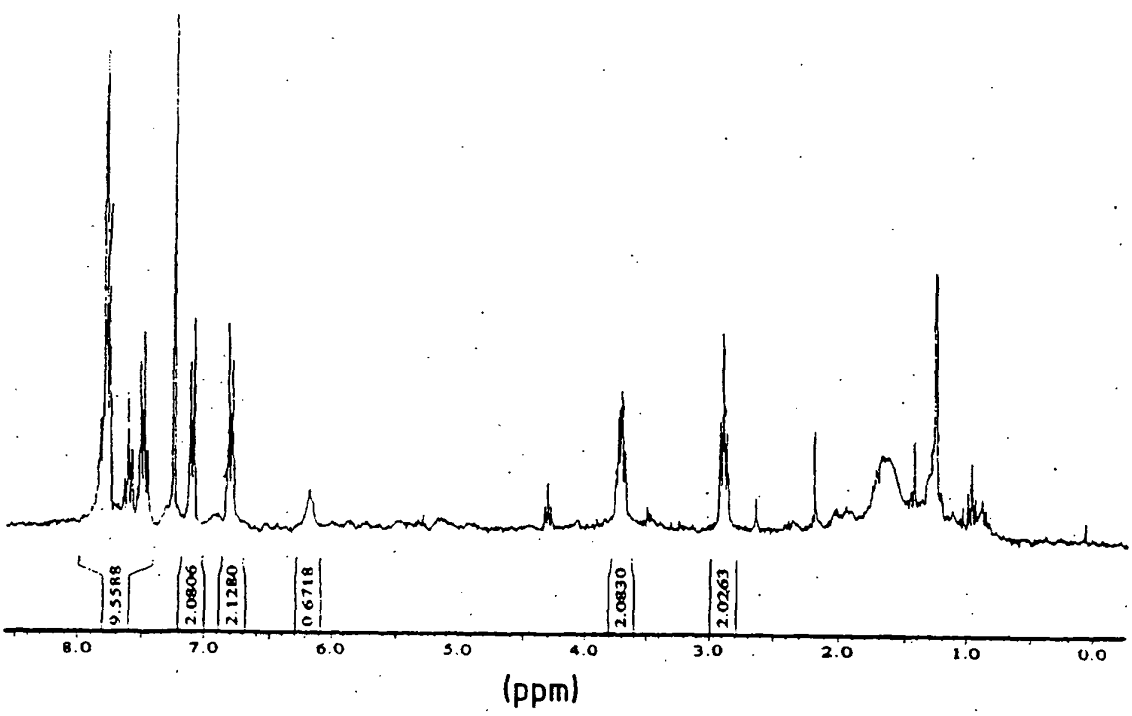
9.5588
2.0806
2.1280
0.6718
2.0830
2.0263
8.0
7.0
6.0
5.0
4.0
3.0
2.0
1.0
0.0
(ppm)

FIG.21

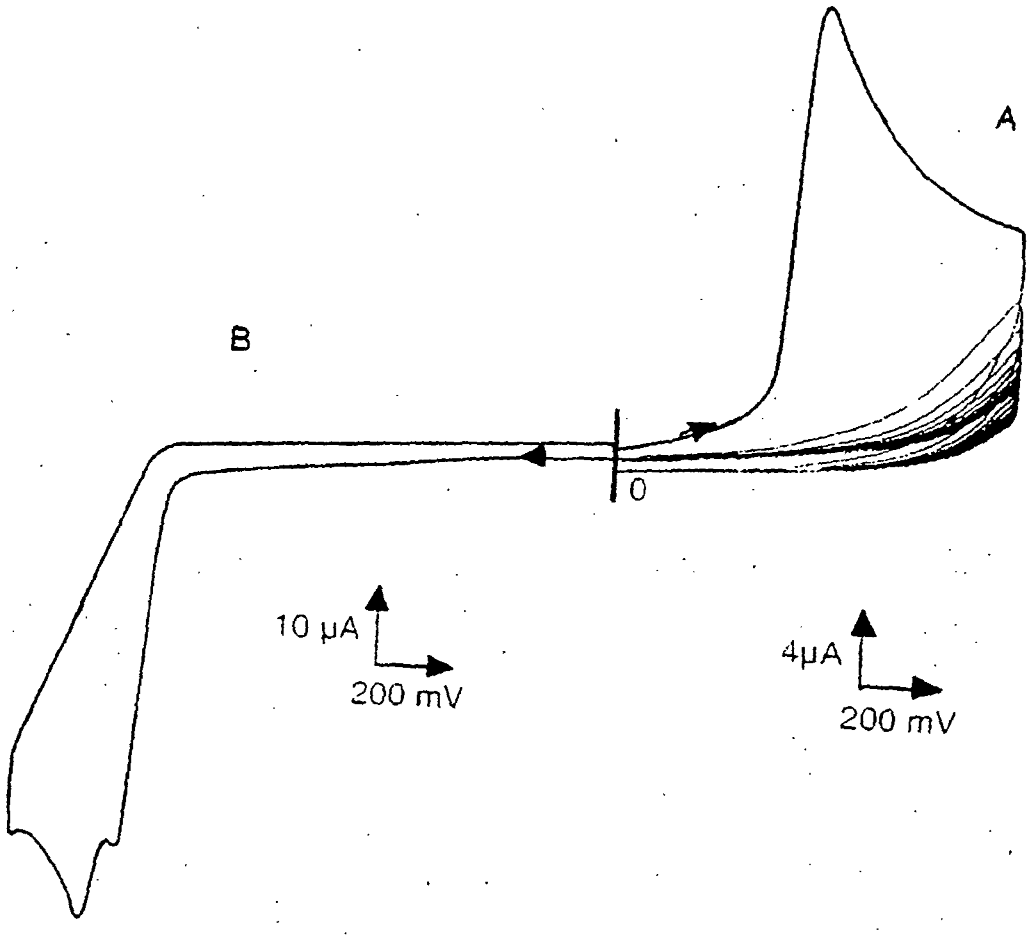


FIG.22

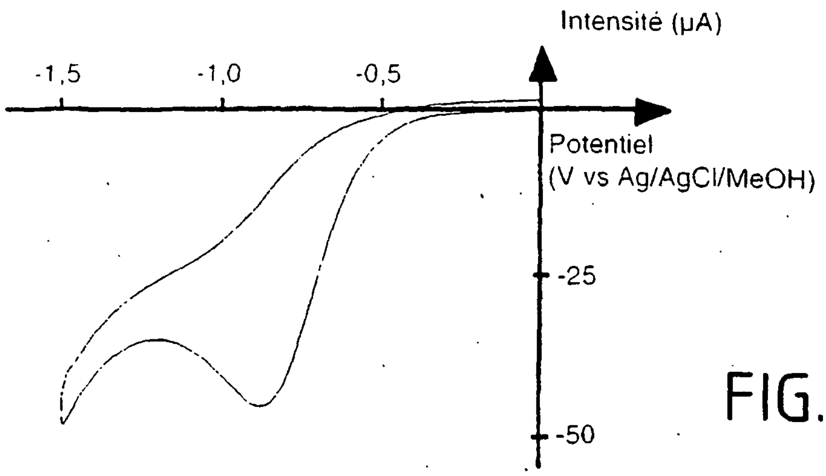


FIG.23

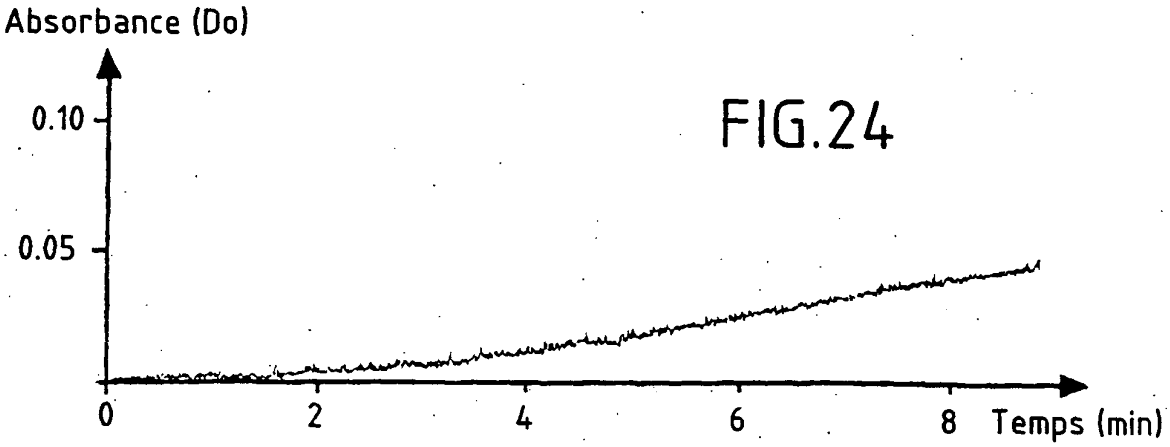


FIG.24

FIG.25

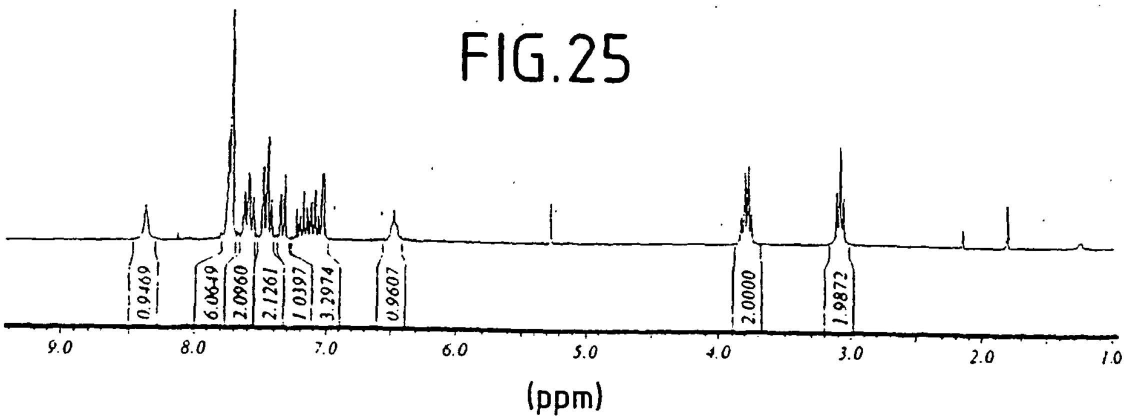

FIG.26

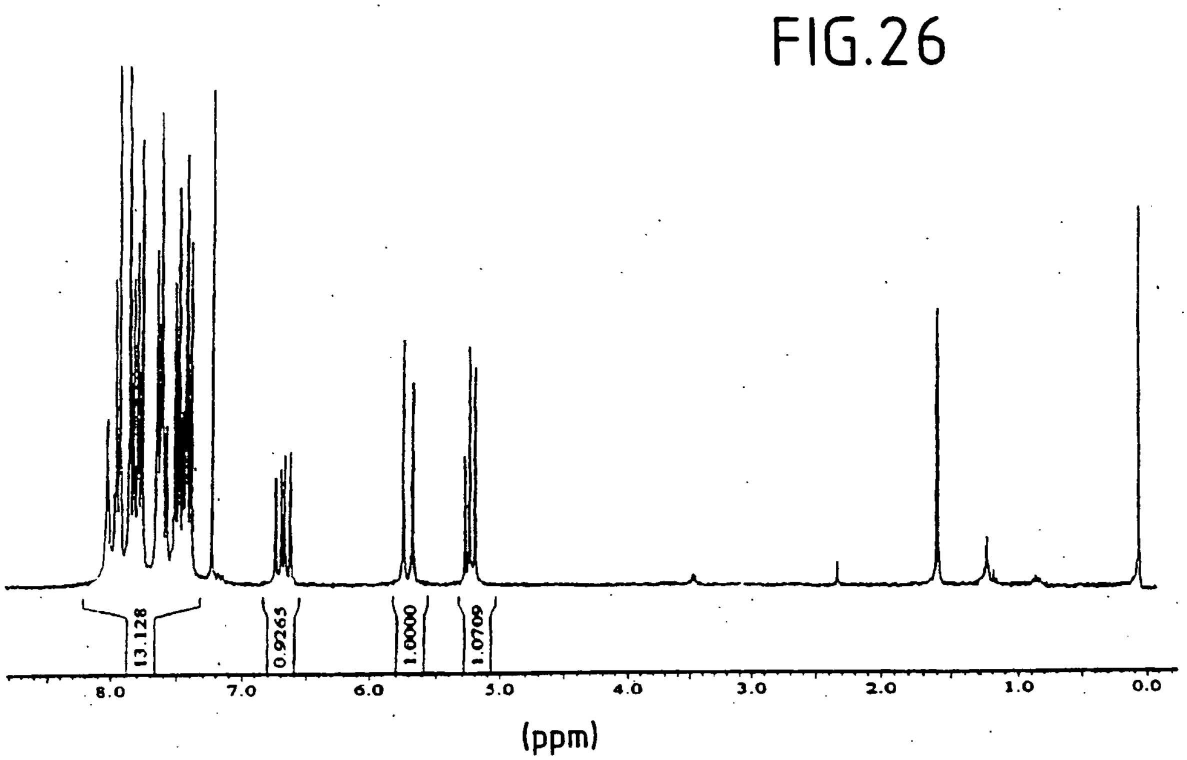
13.128
0.9265
1.0000
1.0709
8.0
7.0
6.0
5.0
4.0
3.0
2.0
1.0
0.0
(ppm)

FIG.27

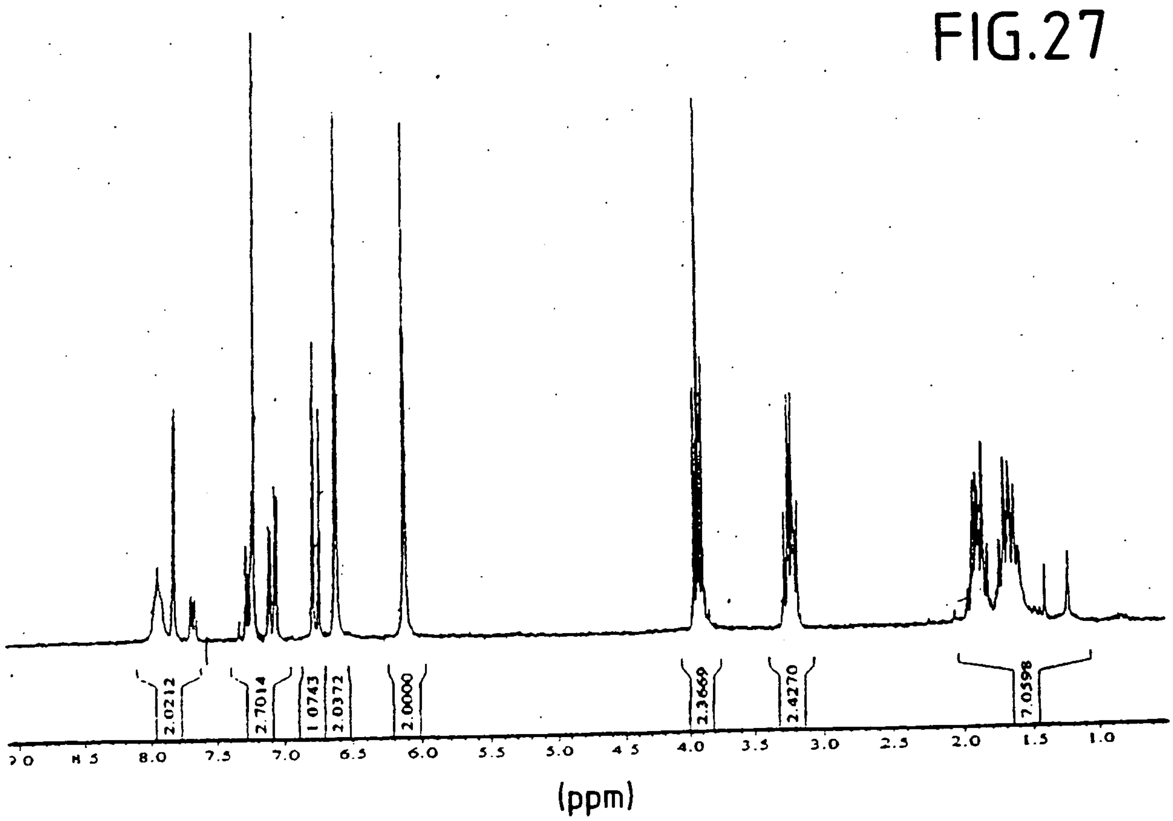
2.0212
2.7014
1.0743
2.0372
2.0000
2.3669
2.4270
7.0598
8.0
7.5
7.0
6.5
6.0
5.5
5.0
4.5
4.0
3.5
3.0
2.5
2.0
1.5
1.0
(ppm)

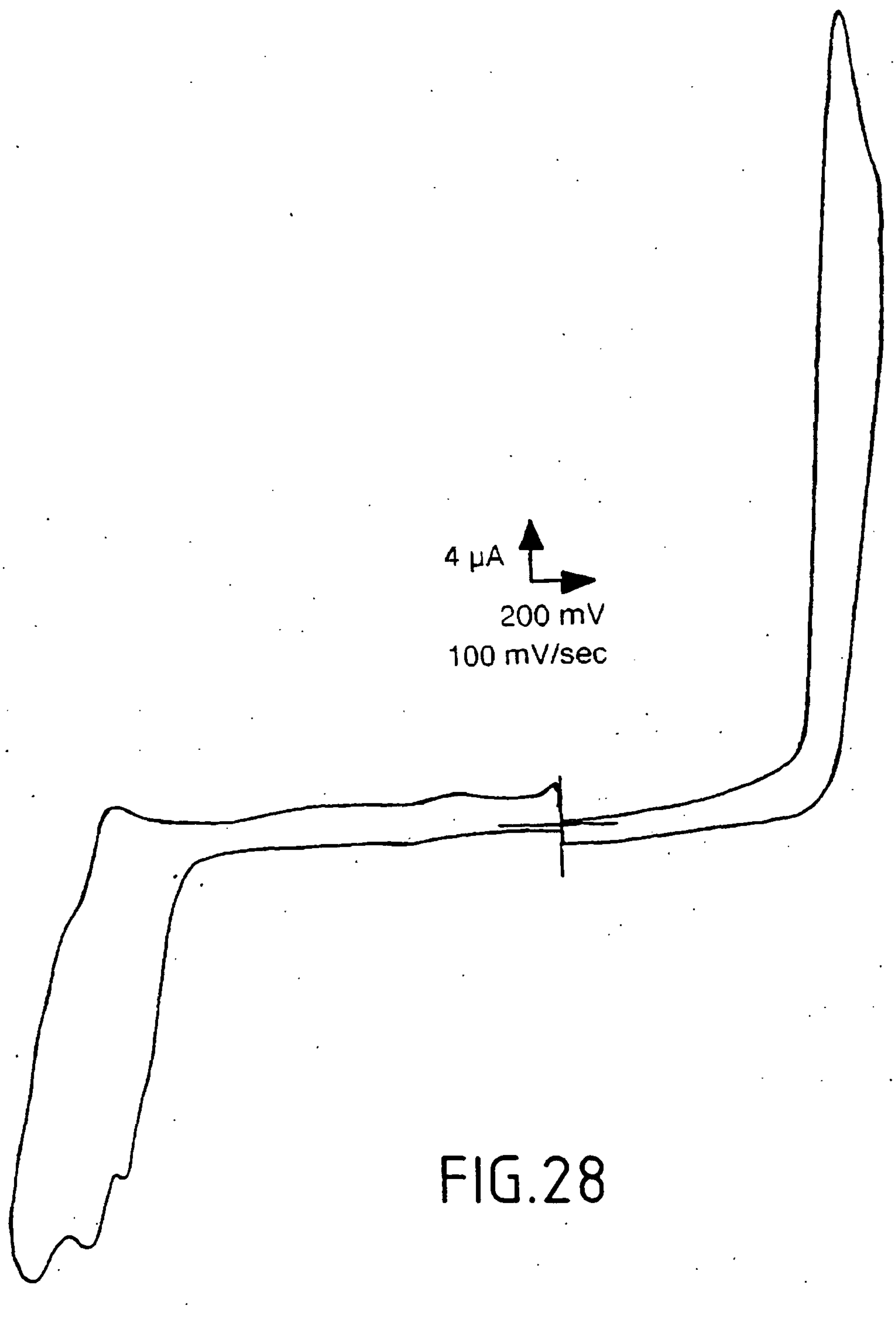


FIG.28

FIG.29

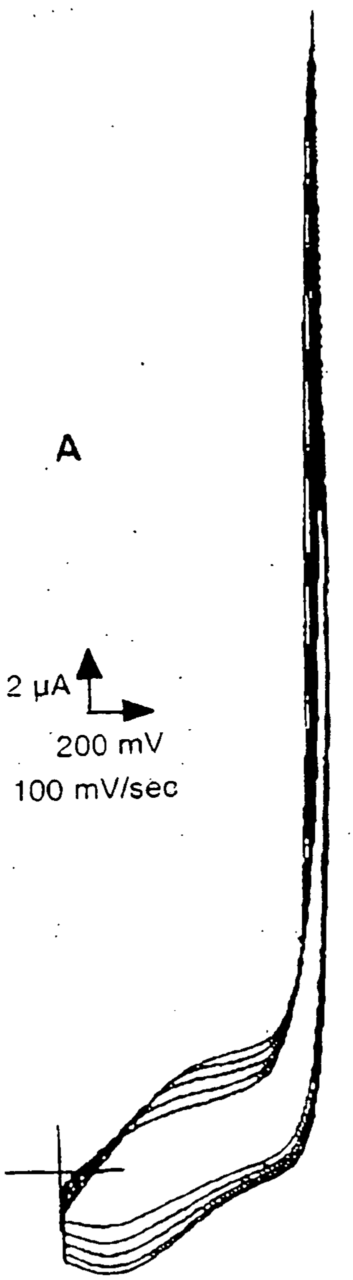


FIG.30

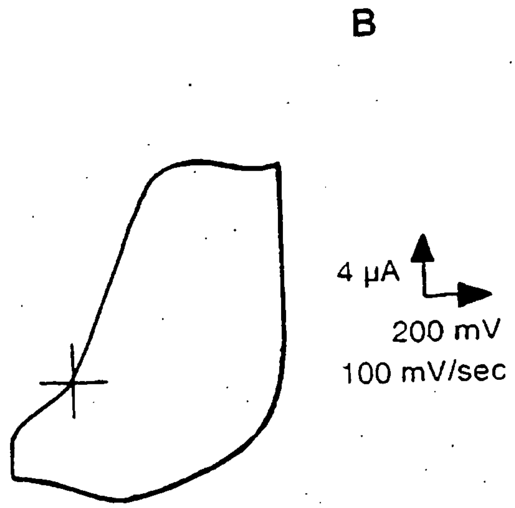

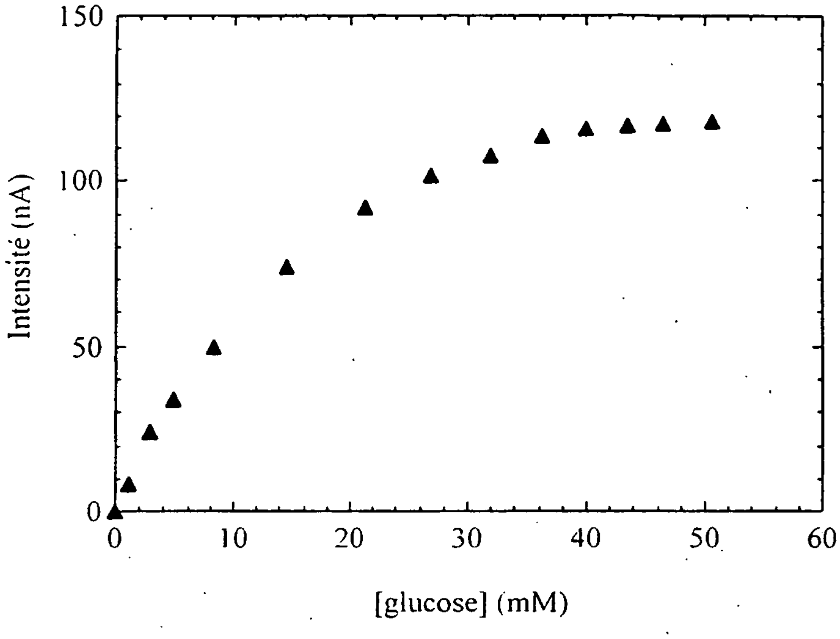
150
100
50
0
Intensité (nA)
0
10
20
30
40
50
60
[glucose] (mM)

FIG. 31

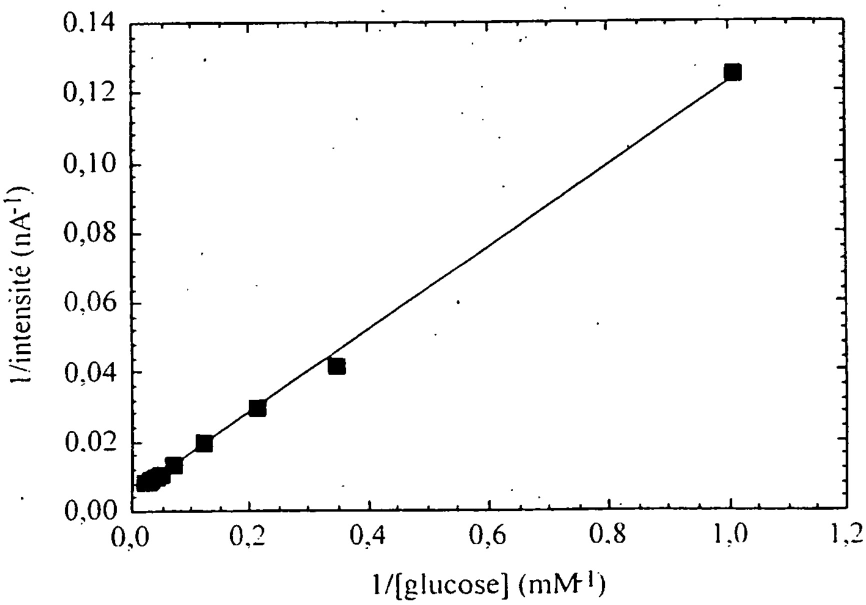
0,14
0,12
0,10
0,08
0,06
0,04
0,02
0,00
1/intensité (nA-1)
0,0
0,2
0,4
0,6
0,8
1,0
1,2
1/[glucose] (mM-1)

FIG. 32

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9210092 A
- FR 2703359 A
- FR 2750136 A
- EP 314009 A
- WO 9010655 A
- FR 2720832 A


### Littérature non-brevet citée dans la description


- **L.F. ROZSNYAI ; D.R. BENSON ; S.P.A. FODOR ; P.G. SCHULTZ.** *Angew, Chem. Int. Ed. Engl.,* 1992, vol. 31, 759
- **SERGE COSNIER.** *Can J. Chem. Eng.,* 1998, vol. 76, 1000